(19) Europäisches Patentamt

European Patent Office

Office européen des brevets

(11) Numéro de publication: **0 192 001 B1**

(12) **FASCICULE DE BREVET EUROPEEN**

(45) Date de publication de fascicule du brevet: **11.03.92**    (51) Int. Cl.⁵: **C12Q 1/68**, C12N 15/37

(21) Numéro de dépôt: **85402362.9**

(22) Date de dépôt: **29.11.85**

(54) **Sondes à papillomavirus et procédé de diagnostic in vitro d'infections à papillomavirus.**

(30) Priorité: **30.11.84 FR 8418369**
        **09.05.85 FR 8507073**

(43) Date de publication de la demande:
    **27.08.86 Bulletin  86/35**

(45) Mention de la délivrance du brevet:
    **11.03.92 Bulletin  92/11**

(84) Etats contractants désignés:
    **AT BE CH DE FR GB IT LI LU NL SE**

(56) Documents cités:

    **PROC. NATL. ACAD. SCI., vol. 81, no. 25, décembre 1984, pages 7880-7884; J.T. SCHILLER et al.: "Identification of a second transforming region in bovine papillomavirus DNA"**

(73) Titulaire: **INSTITUT PASTEUR**
    **25/28, rue du Docteur Roux**
    **F-75015 Paris(FR)**

    Titulaire: **INSTITUT NATIONAL DE LA SANTE ET DE LA RECHERCHE MEDICALE (INSERM)**
    **101, rue de Tolbiac**
    **F-75654 Paris Cédex 13(FR)**

(72) Inventeur: **Orth, Gérard**
    **49, rue du Dr. Roux**
    **F-92330 Sceaux(FR)**
    Inventeur: **Favre, Michel**
    **7, rue Guillout**
    **F-75015 Paris(FR)**
    Inventeur: **Kremsdorf, Dina**
    **35, rue Esquirol**
    **F-75013 Paris(FR)**
    Inventeur: **Croissant, Odile**
    **19, rue Auguste Lançon**
    **F-75013 Paris(FR)**
    Inventeur: **Pehau-Arnaudet, Gérard**
    **25, avenue Paul Signac**
    **F-93100 Montreuil(FR)**
    Inventeur: **Beaudenon, Sylvie**
    **108, avenue Foch Montry**
    **F-77450 Esbly(FR)**

CHEMICAL ABSTRACTS, vol. 102, no. 3, 21 janvier 1985, page 190, abrégé no. 18873w, Columbus, Ohio, US; D. KREMSDORF et al.: "Molecular cloning and characterization of the genomes of nine newly recognized human papillomavirus types associated with epidermodysplasia verruciformis" & J. VIROL. 1984, 52(3), 1013-18

CHEMICAL ABSTRACTS, vol. 102, no. 3, 21 janvier 1985, page 190, abrégé no. 18874x, Columbus, Ohio, US; A. GASSENMAIER et al.: "Molecular cloning and characterization of the DNAs of human papillomaviruses 19, 20 and 25 from a patient with epidermodysplasia verruciformis" & J. VIROL. 1984, 52(3), 1019-23

CHEMICAL ABSTRACTS, vol. 98, no. 19, 9 mai 1983, page 243, abrégé no. 157580k, Columbus, Ohio, US; R. OSTROW et al.: "Characterization of two HPV-3 related papillomaviruses from common warts that are distinct clinically from flat warts or epidermodysplasia verruciformis" & J. INVEST. DERMATOL. 1983, 80(5), 420-4

NATURE, vol. 299, 7 octobre 1982, pages 529-534, MacMillan Journals Ltd., US; E.Y. CHEN et al.: "The primary structure and genetic organization of the bovine papillomavirus type 1 genome"

Journal of virology, 1982, p. 436-447

Journal of virology, 1983, p. 340-351

(74) Mandataire: **Gutmann, Ernest et al**
**S.C. Ernest Gutmann - Yves Plasseraud 67, boulevard Haussmann**
**F-75008 Paris(FR)**

## Description

L'invention concerne des ADNs de papillomavirus, et plus particulièrement des sondes dérivées de ces papillomavirus, ainsi que des procédés les mettant en oeuvre pour le diagnostic in vitro d'infections à papillomavirus.

L'expression "papillomavirus" recouvre un grand nombre de virus ayant en commun d'être tenus pour responsables de plusieurs formes d'infections virales s'étageant entre les verrues cutanées ou de muqueuses relativement bénignes et des hyperplasies susceptibles de dégénérer en néoplasies intra-épithéliales et en cancers cutanés. Parmi des infections à papillomavirus, on mentionnera également plus particulièrement les épidermodysplasies verruciformes, qui seront quelquefois ci-après désignées sous l'expression "EV".

Un certain nombre de types de papillomavirus a déjà été décrit. Dans le cadre de la présente demande de brevet seront décrits de nombreux types et sous-types nouveaux de papillomavirus qui ont été isolés à partir de verrues ou lésions maculaires disséminées, susceptibles de donner lieu au développement précoce de cancers de la peau chez d'importantes proportions des malades qui en sont affectés.

Des études récentes ont révélé l'importance de facteurs immunitaires et le rôle majeur de divers types de virus de papillomes humains (HPV), ces facteurs s'ajoutant au rôle déjà décrit dans la littérature de facteurs génétiques divers et des rayonnements actiniques dans la pathogénèse des infections aux papillomavirus.

L'invention découle des observations qui ont pu être faites quant aux comportements relatifs d'un grand nombre de papillomavirus nouvellement isolés, dont les caractéristiques génomiques essentielles seront définies ci-après.

L'étude d'un petit nombre de cas d'EV avait déjà conduit à la caractérisation de six types d'HPV après clonage moléculaire de leurs génomes (KREMSDORF, D. et al, 1982, J. Virol. 43:436-447 et KREMSDORF et al, 1983, J. Virol. 48:340-351). Ces HPV ont été divisés en trois groupes en fonction de l'absence d'hybridation croisée ou d'une hybridation croisée très faible entre les génomes appartenant à des groupes différents. Le premier groupe comprenait les HPV3a et 10 qui sont associés aux verrues planes observées chez certains malades atteints d'EV et dans la population générale ; des séquences d'ADN apparentées à celles de l'HPV3a ont été trouvées dans un cancer de malade atteint d'EV. Le second groupe comprenait les HPV5, 8 et 12, les génomes des HPV5 et 8 ayant été détectés dans des cancers de malades atteints d'EV. Le troisième groupe est constitué à ce jour d'un seul virus, l'HPV9. A l'exception d'un receveur d'une allogreffe rénale présentant une immunosuppression, qui s'était révélé être infecté par l'HPV5, les virus des deux derniers groupes n'avaient été détectés que chez des malades atteints d'EV, la plupart d'entre eux étant infectés par plusieurs virus. Il faut noter que parmi les 14 types d'HPV actuellement mentionnés dans la littérature (références bibliographiques 1-5, 8,9, 13, 14, 16, 18-20 indiquées plus loin), quatre se sont révélés associés spécifiquement à l'EV qui est une maladie rare.

Les papillomavirus suivants : HPV 14a, 14b, 15, 17a, 17b, 19, 20, 21, 22, 23 et 24 sont décrits dans l'article de KREMSDORF et autres auteurs dans "Journal of Virology, Dec. 1984, pages 1013-1018".

Les travaux qui ont conduit à l'invention et qui ont permis d'isoler un nombre important de nouveaux types et sous-types de papillomavirus permettent désormais d'envisager des techniques de diagnostic in vitro plus affinées. Plus particulièrement, l'invention fournit des techniques perfectionnées d'identification de papillomavirus, par exemple obtenus à partir de lésions ou de coupes de biopsies et permet de faire des diagnostics plus précis, dont pourront également résulter des pronostics améliorés quant à l'évolution possible des lésions en cause.

D'une façon générale, il sera remarqué que les papillomavirus, bien que très différents entre eux, ont des tailles de l'ordre de 7000-8000 paires de bases. En outre leurs génomes peuvent néanmoins présenter certains degrés d'homologie. Dans ce qui suit, on fera référence à des évaluations des pourcentages d'homologies entre types et sous-types de papillomavirus, ces pourcentages d'homologies résultant d'essais d'hybridation réalisés dans des conditions dites non stringentes ou non strictes ou encore dans des conditions d'hybridation stringente ou stricte.

Parmi les papillomavirus, on distinguera plusieurs types de papillomavirus, ceux-ci se distinguent par leurs pourcentages d'homologies mesurés dans des conditions strictes ou stringentes. Il sera dit que les papillomavirus qui, dans ces dernières conditions, présentent des pourcentages d'homologie inférieurs à 50 % appartiennent à des types différents. On notera à cet égard que les pourcentages d'homologie entre des virus de types différents peuvent même tomber à zéro dans lesdites conditions strictes ou stringentes. Les virus pour lesquels on observe, dans ces conditions strictes ou stringentes, des pourcentages d'homologie supérieurs à 50 % sont considérés comme appartenant au même type et former des sous-types différents au sein de ce même type.

Les essais d'hybridation dans les conditions non strictes ou non stringentes impliquent la mise en contact mutuelle d'ADNs provenant de deux isolats de virus dans les conditions suivantes decrites par HEILMAN C.A. et al, 1980, J. Virol., 36, 395-407, et CROISSANT et al, 1982, C.R. Acad. Sc. Paris, 294, 581-586 (molécules hétéroduplexes).

Les essais d'hybridation dans les conditions strictes ou stringentes impliquent la mise en contact mutuelle d'ADNs provenant de deux isolats de virus dans les conditions décrites par KREMSDORF, D. et al. ((1982), J. Virol. 43:436-447 et 1983, J. Virol. 48:340-351) et DAVIS R.W. et al., 1971, Methods Enzymol., 21, 413-428 (molécules hétéroduplexes).

Schématiquement, on remarquera que les papillomavirus appartenant à un même type présentent des séquences hybridables ayant des séquences nucléotidiques sensiblement identiques sur 80 à 100 % de la totalité de leurs longueurs respectives, ces séquences homologues peuvent être réduites à 60 %, voire moins chez des papillomavirus de types différents Le degré d'identité ou d'analogie des séquences de papillomavirus de types différents qui s'hybrident mutuellement dans des conditions non strictes ou non stringentes, peut évidemment être plus faible que dans le cas de papillomavirus appartenant au même type.

L'étude à laquelle les inventeurs ont procédé a montré à la fois que le degré d'hétérogénéité génétique entre papillomavirus de divers types était plus important que ce qui était apprécié auparavant et en même temps que les différents types se trouvaient souvent associés à des formes ou variantes d'infections présentant un certain degré de spécificité.

L'invention concerne par conséquent non seulement les ADNs susceptibles d'être isolés à partir des différents papillomavirus nouveaux qui ont été isolés et les sondes qui peuvent être constituées de tout ou partie de ces ADNs, mais encore des mélanges ou "cocktails" de types de papillomavirus susceptibles d'être mis en oeuvre plus efficacement pour le diagnostic de diverses catégories d'infection, voire des niveaux de risques qu'accompagnent la découverte chez un patient de papillomavirus déterminés. Le nombre des sondes à papillomavirus décrites dans la présente demande, auxquelles s'ajoutent, le cas échéant, celles constituées à partir des ADNs génomiques de papillomavirus déjà isolés précédemment et leurs associations dans des mélanges déterminés, permettrait donc la réalisation de diagnostics plus affinés, notamment une plus grande discrimination des diverses catégories d'infections imputables aux divers types de papillomavirus ou susceptibles de se développer sous l'effet de ces derniers types et, au sein d'une catégorie d'infections déterminées, de mieux pronostiquer le degré de risque que ces dernières se transforment en des maladies plus redoutables. Par exemple l'invention a pour but de fournir des moyens permettant, dans le cas des infections se manifestant par des épidermodysplasies verruciformes, de mieux apprécier le degré de risque que ces dernières évoluent vers des cancers cutanés.

D'une façon générale et dans le but de simplifier l'exposé qui suit, les génomes entiers des papillomavirus seront désignés par l'abréviation "ADN-HPV".

Dans le même but de simplification, il est fait référence dans ce qui suit aux dessins, dans lesquels les figures consistent en des cartes physiques de restriction d'ADN-HPVs, parmi lesquelles d'ailleurs des ADN-HPVs de papillomavirus déjà connus.

Les cartes physiques donnent la position de sites de coupure par diverses endonucléases de restriction. L'origine des cartes est généralement constituée par un site unique de coupure. Les distances à l'origine sont exprimées en pourcentage de longueur de génome. Une unité de carte représente 1 % de longueur de génome.

L'invention concerne tout d'abord plus spécifiquement chacun des ADN-HPVs choisis parmi l'ensemble des ADNs qui présentent des tailles qui s'étagent entre 7000 et 8000 paires de bases choisies parmi les ADNs clonés déposés le 30 novembre 1984 à la C.N.C.M. (Collection Nationale de Cultures de Microorganismes de l'INSTITUT PASTEUR de Paris), sous les numéros ci-après :

| HPV 2d | n° I-379 |
| HPV 10bA | n° I-380 |
| HPV 10bB | n° I-381 |
| HPV 28 | n° I-394 |
| HPV 29 | n° I-395 |
| HPV 31 | n° I-396 |
| HPV 32 | n° I-397 |
| IP-2 | n° I-450 |
| IP-4 | n° I-449 |

L'invention concerne également des fragments des ADN HPVs précédents ou capables de s'hybrider avec ceux-ci. notamment dans des conditions strictes. De même, elle concerne les ADNs recombinants contenant tout ou partie de chacun des ADN-HPVs sus-indiqués, et plus particulièrement des ADNS recombinants contenant des fragments correspondant aux gènes E1, E6-E7, L1 et L2 respectivement ou encore des fragments contenant des séquences correspondant aux régions intergéniques desdits ADN-HPVS. Elle concerne enfin les sondes qui peuvent être constituées à partir de ces ADN-HPVs respectifs ou à partir des fragments correspondants et les procédés de diagnostic in vitro mettant en jeu lesdites sondes.

Les cartes de restriction des ADN-HPVs obtenus à partir des papillomavirus et qui répondent aux désignations HPV-2d, HPV 10b, HPV-14A, HPV-14B, HPV-15, HPV-17a, HPV-17b, HPV-19, HPV-20, HPV-21, HPV-22, HPV-23, HPV-24, HPV-28, HPV-29, HPV-31 et HPV-32, HPV-IP2 et HPV-IP4, apparaissent dans les dessins.

Les préparations d'ADN viral ont été extraites sélectivement (LUTZNER, M.A. et al., 1983, Lancet ii:422-424) à partir de produits de grattage de lésions bénignes de six malades européens atteints d'EV et de deux malades sud-américains atteints d'EV. Les ADNs d'HPV ont été purifiés par centrifugation en équilibre dans des gradients de chlorure de césium et/ou sédimentation dans des gradients de saccharose en présence de bromure d'éthidium, selon des modes opératoires précédemment décrits (articles de KREMSDORF, D. et al. déjà décrits et ORTH, G. et al., 1980, Cold Spring Harbor Conf. Cell Proliferation 7:259-282). Les préparations d'ADN ont été traitées avec des endonucléases de restriction et les produits de digestion ont été séparés par électrophorèse sur des gels d'agarose (articles de KREMSDORF et al. déjà mentionnés). En plus des HPV5, 8 et 12 (articles de KREMSDORF et al. déjà mentionnés) et de l'HPV2 (HEILMAN, C.A. et al., 1980, J. Virol. 36:395-407 et ORTH, G. et al., 1980, Cold Spring Harbor Conf. Cell Proliferation 7: 259-282) trouvés dans les verrues vulgaires d'un des malades, ont été identifiés onze souches fournissant des modèles majeurs de clivage par des enzymes de restriction d'ADN, différents de ceux des types précédemment caractérisés . Les nouveaux types d'HPV ont reçu un numéro et les sous-types d'un type ont reçu le même numéro suivi d'une lettre, selon l'ordre chronologique de leur identification (COGGIN, J.R. et al., Cancer Res. 39:545-546). Les génomes des 11 nouveaux HPVs ont été clonés dans Escherichia coli K12, souche C600 (article de KREMSDORF, D. et al. (1983) déjà mentionné). Les ADNs ont été insérés sous forme de molécules de longueur unitaire à l'exception de deux fragments d'ADN d'HPV24 produits par l'endonucléase BamHI. Ils ont été insérés soit dans le plasmide pBR322 (SUTCLIFFE, J.G., 1978, Nucleic Acids Res. 5:2721-2728), en utilisant les sites de clivage uniques de Aval, de BamHI et de HindIII, soit dans un plasmide recombinant ayant intégré le fragment HindIII B de l'ADN d'HPV5 (article de KREMSDORF, D. et al., 1982, déjà mentionné), qui contient un site SacI unique. Plus particulièrement les HPV17b et 22 ont été insérés sous forme de molécules d'ADN de longueur unitaire après clivage avec une enzyme (SacI) qui ne clive qu'une fois l'ADN d'HPV et le plasmide recombinant pBR322 contenant le fragment HindIII B de l'DN d'HPV5. L'ADN d'HPV14a a été inséré dans le plasmide pBR322 sous forme d'une molécule d'ADN de longueur unitaire après digestion incomplète de la préparation d'ADN viral avec HindIII, une enzyme qui produit deux fragments de 96,1 et 3,9 % de la longueur du génome. Les fragments BamHI A et B d'HPV24 (ayant des tailles correspondant à respectivement 83,1 et 16,9 % de la longueur du génome) ont été insérés séparément dans le plasmide pBR322.

Les clones isolés et les sources des HPV correspondants résultent du tableau I ci-après :

TABLEAU 1. ORIGINE DES ADNs d'HPV CLONES

| Malade[a] | Nationalité | Source[b] | Type d'ADN d'HPV cloné | Enzyme[c] de clonage | Autres types d'HPV trouvés chez les malades |
|---|---|---|---|---|---|
| 1 | polonaise | verrues ; genoux | 14a | Hind III | 5 |
| 2 | française | verrues ; mains | 15 | Bam HI | |
| 3 | colombienne | macules ; tronc | 14b | Bam HI | |
| 4 | italienne | macules ; poitrine | 17a | Bam HI | 5 |
| | | | 17b | Sac I | 5 |
| 5 | hollandaise | macules ; dos | 22 | Sac I | 5,8,17a |
| | | macules ; poitrine | 19 | Bam HI | |
| 6 | colombienne | verrues ; mains | 24 | Bam HI | 5,8,24 |
| 7 | polonaise | verrues ; genoux | 20 | Ava I | 2,12,17a,20 |
| 8 | polonaise | macules ; avant-bras | 21 | Bam HI | 5,8,20 |
| | | | 23 | Bam HI | |

Pour identifier les plasmides recombinants, les mobilités électrophorétiques des produits de digestion des ADNs recombinants et des ADNs d'HPV non clonés ont été comparées après traitement avec un mélange de deux endonucléases de restriction comprenant l'endonucléase utilisée pour l'insertion des séquences virales dans le plasmide. Le nombre et la taille des fragments isolés ont indiqué que dans chaque cas les génomes viraux entiers ont été intégrés. Une hétérogénéité des tailles des ADNs a été observée lorsque les ADNs des HPVs, non clonés ou excisés des séquences plasmidiques, ont été analysés par électrophorèse sur gel d'agarose (données non présentées). Les ADNs d'HPV14b, 19, 20 et 21 ont des tailles semblables à ceux des HPV3a, 5, 8 et 12 (environ 7700 paires de nucléotides (articles de KREMSDORF, D. déjà mentionnés), tandis que les ADNs d'HPV15, 17a, 17b, 22 et 23 ont des tailles plus faibles semblables à celle d'HPV9 (environ 7200 paires de nucléotides) (articles de KREMSDORF, D., 1982) et ORTH, G., 1980, déjà mentionnés).

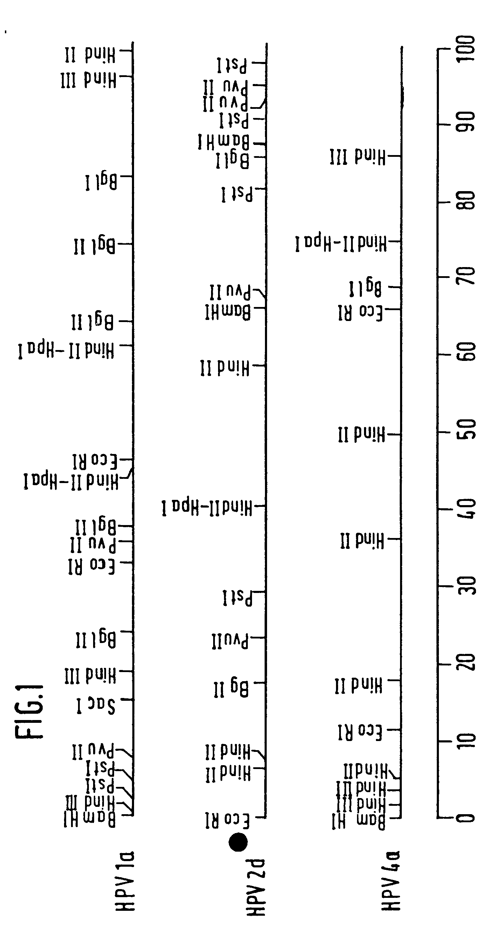

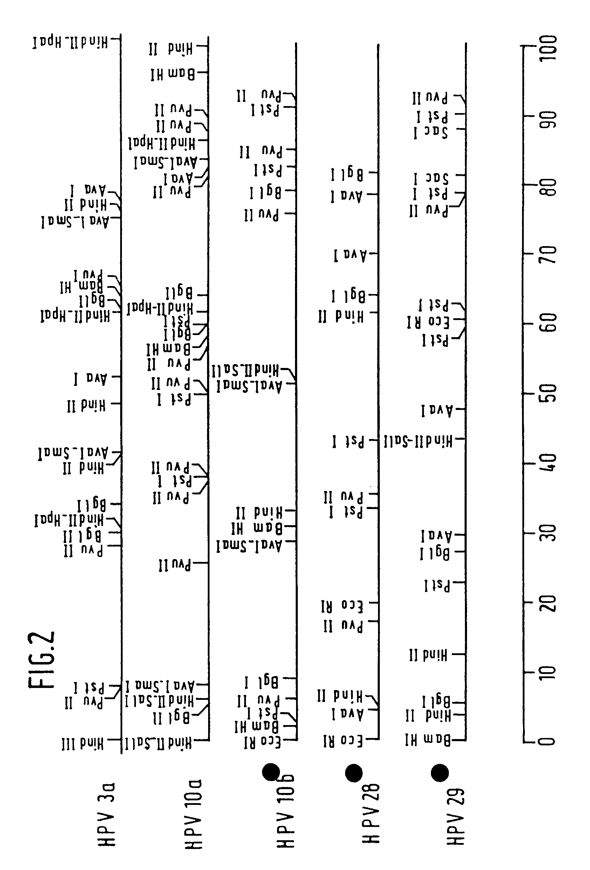

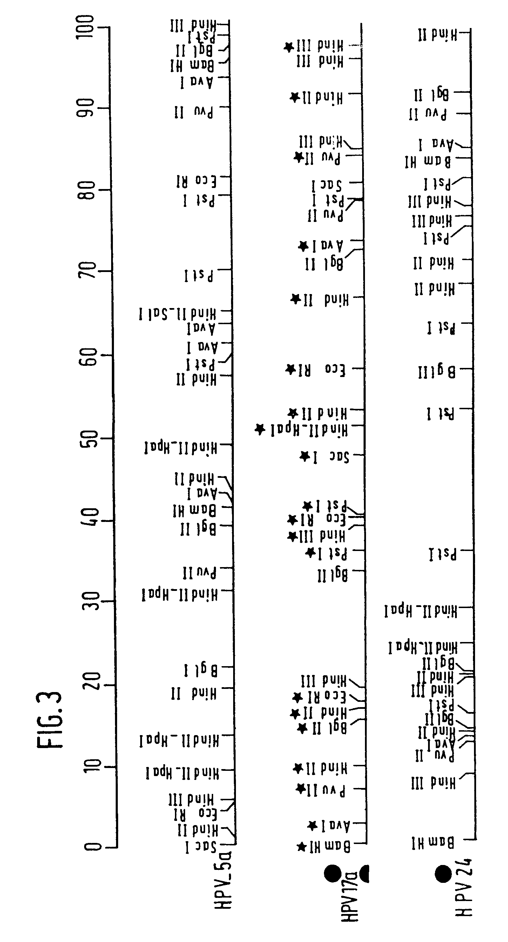

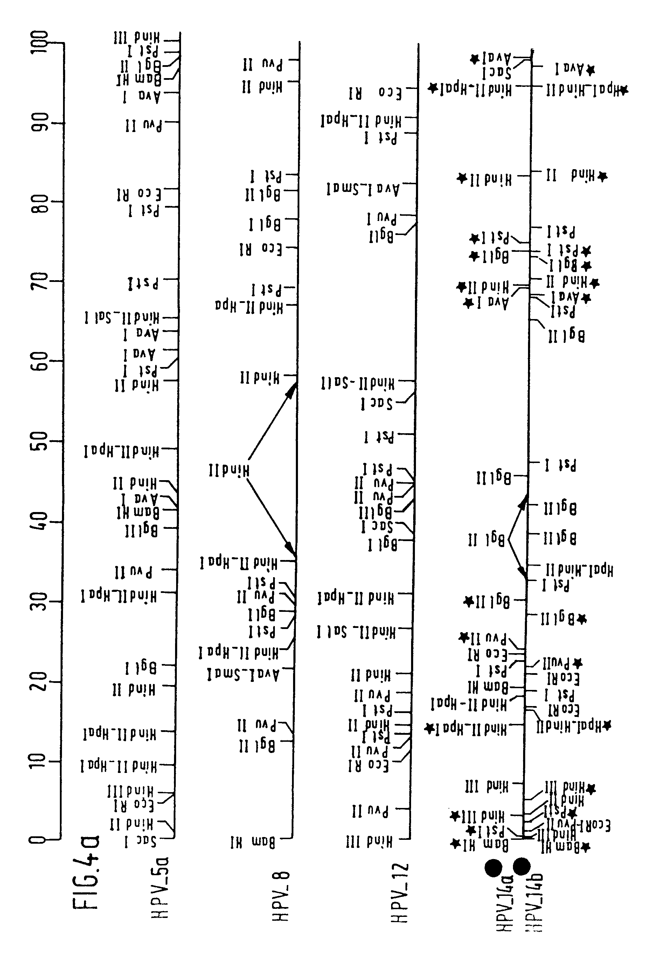

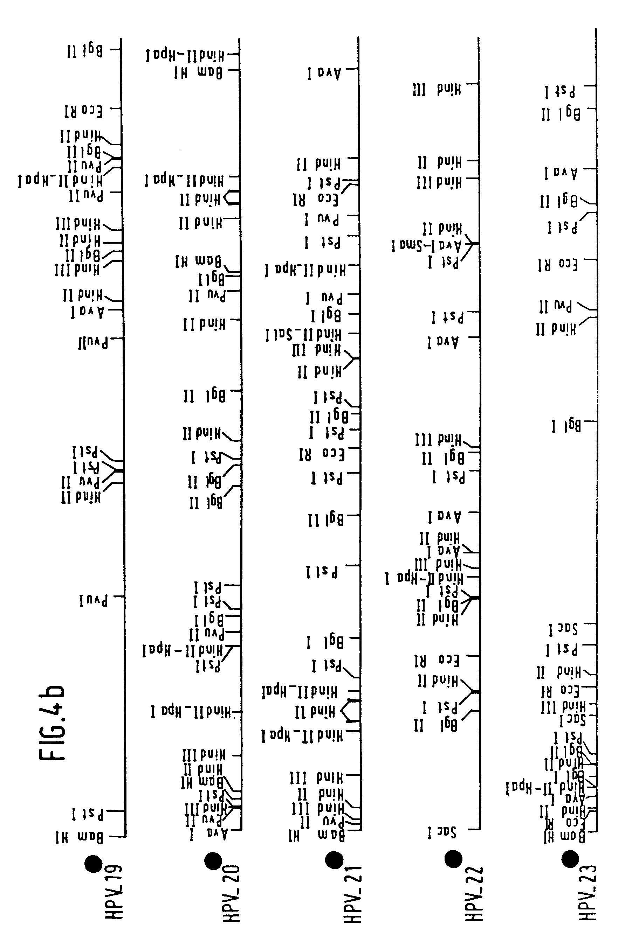

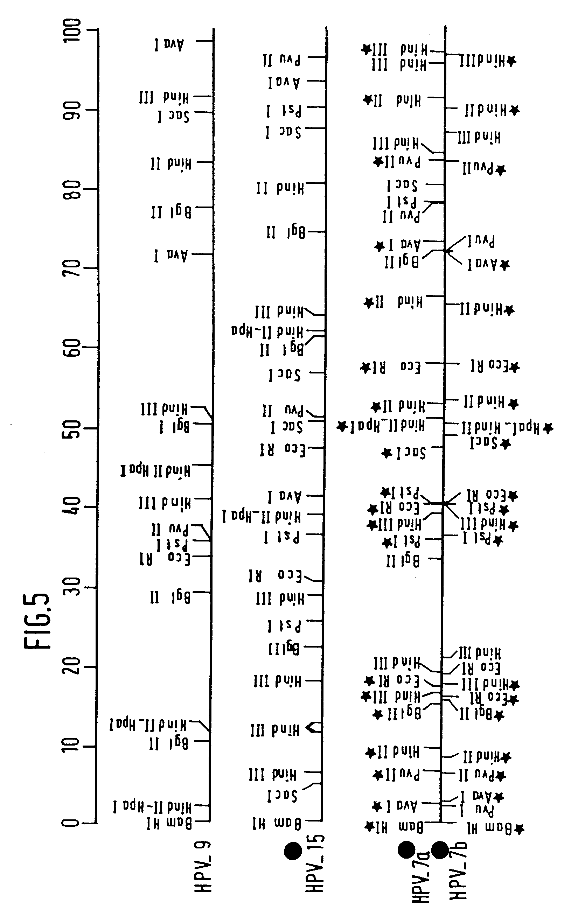

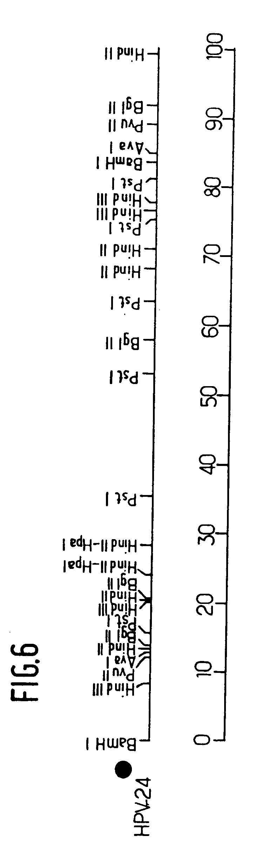

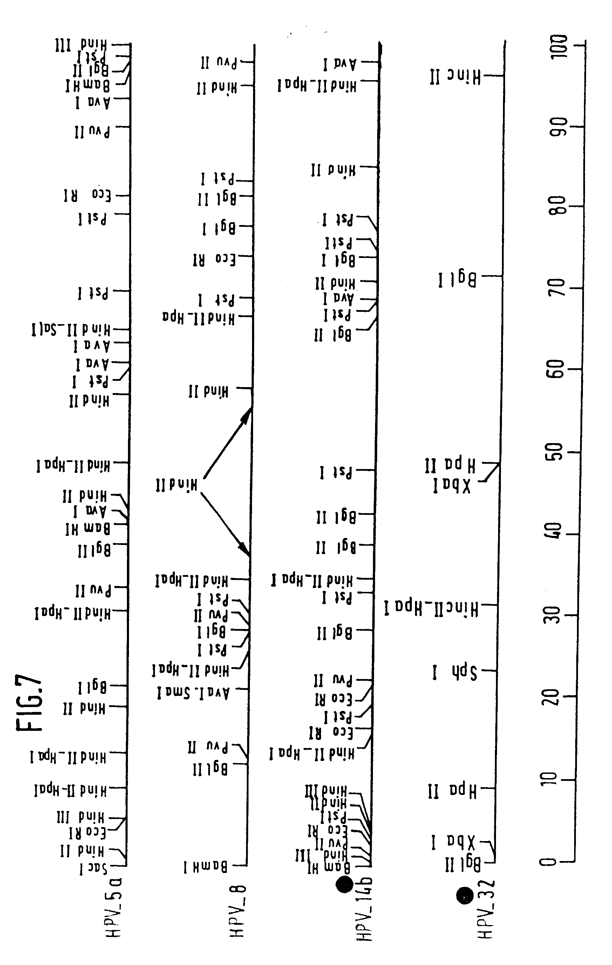

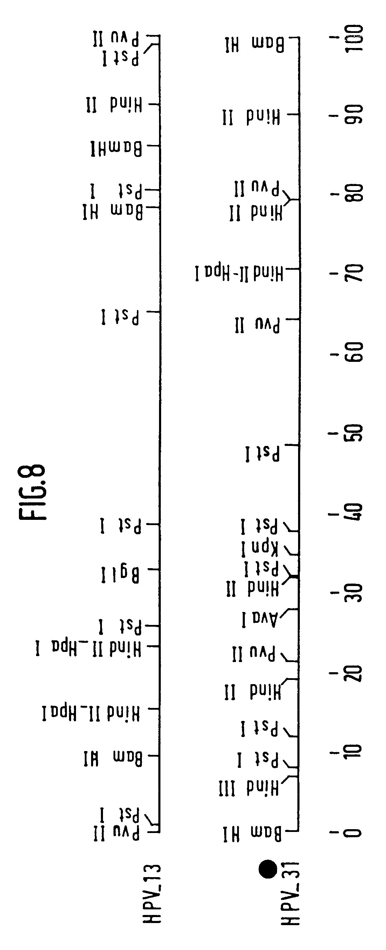

La sensibilité des génomes viraux clonés à 14 endonucléases de restriction a été analysée et les cartes physiques ont été établies (figures 1 à 10). Les cartes de restriction de certains des ADN-HPVs sont répétées dans certaines des figures pour les motifs exposés plus loin. Entre 22 et 33 sites de clivage ont été localisés selon les méthodes précédemment décrites (9). Aucune analogie évidente n'a été détectée entre ces cartes, à l'exception de celles des HPV14a et 14b, d'une part (figs.4a et 4b) et entre celles des HPV17a et 17b, d'autre part (figure 5), Parmi les 21 et 31 sites localisés respectivement sur les ADNs des HPV14a et 14b, 15 se sont révélés communs lorsque l'un des deux sites de clivage BamHI de l'ADN d'HPV14a a été aligné avec le site de clivage BamHI unique de l'ADN d'HPV14b. De façon semblable, 21 des 29 sites de clivage situés sur l'ADN d'HPV17a ont également été trouvés sur l'ADN d'HPV17b (avec 26 sites), lorsque les sites de clivage BamHI uniques ont été alignés.

Aucune analogie évidente n'a été détectée entre ces cartes et celles précédemment établies pour les HPV associés à l'EV (HPV3a, 5, 8, 9, 10 et 12) (8,9, 16, 18, 20), aux verrues cutanées (HPV1, 2, 4 et 7), et aux lésions des membranes mucocutanées ou muqueuses (HPV6b, 11a, 13 et 16) (1, 33, 19), à l'exception de la carte d'HPV14a qui est étroitement apparentée à la carte d'un HPV isolé d'un malade japonais atteint d'EV (24). Ce dernier isolat diffère de l'HPV14a par un site BamHI et un site HindII additionnels, alors que les localisations des sites AvaI, BamHI, BgII, EcoRI, HindII et HindIII sont semblables dans les deux virus. Des expériences d'hybridation croisée ont confirmé que ces deux virus étaient très étroitement apparentés.

On remarquera encore que quelques sites (ceux indiqués par les flèches) n'ont pas été localisés. Les sites de clivage différant de moins de 2 % de la longueur du génome par leur localisation sont considérés comme conservés (*). Les enzymes ne produisant pas de coupure ont été PvuI, Sal I et SmaI pour les ADNs d'HPV14a et 23 ; PvuI, SacI, SalI et SmaI pour l'ADN d'HPV14b : BgII, PvuI, SalI et SmaI pour les ADNs d'HPV15, 17a et 17b : BgII, SacI, SalI et SmaI pour l'ADN de HPV19 : EcoRI, PvuI, SacI et SmaI pour l'ADN d'HPV20 : Sac I et SmaI pour l'ADN d'HPV21 ; BamHI, BgII, PvuI, PvuII et SalI pour l'ADN d'HPV22 : BgII, EcoRI, PvuI, SacI, SalI et SmaI pour l'ADN d'HPV24.

L'existence d'homologies de séquence entre les ADNs des ADNs d'HPV nouvellement caractérisés ainsi qu'entre ces derniers et les ADNs d'HPV d'EV précédemment caractérisés (HPV3a, 5, 8, 9, 10 et 12) d'HPV associés aux verrues cutanées (HPV1, 2, 4 et 7), et d'HPV associés à des lésions des membranes muqueuses (HPV6b, 11a, 13 et 16) a été étudiée. Des expériences d'hybridation par fixation sur un papier-filtre et d'hybridation ADN-ADN en phase liquide à saturation suivie d'une digestion par la nucléase S1 ont été effectuées dans des conditions strictes ou stringentes précédemment décrites (8, 9). En particulier les ADNs d'HPV ont été marqués par translation de coupure ("nick-translation") et fractionnés par sédimentation dans des gradients de saccharose alcalins (5 à 20 %) comme précédemment décrit (13). Les ADNs d'HPV marqués (4000 cpm) ont été incubés dans du NaCl 0,48 M-EDTA 1 mM (pH 6,8) à 68°C, en présence soit d'ADN de thymus de veau (20 μg), soit d'ADN d'HPV non marqué (0,20 μg) comme précédemment décrit (8, 9). Les activités spécifiques des sondes d'ADN d'HPV ont varié entre $5,3 \times 10^7$ et $2 \times 10^8$ cpm/μg. Le pourcentage d'hybridation a été déterminé par mesure des fractions résistant à la nucléase S1. Les nombres représentent les valeurs corrigées pour l'autorenaturation spontanée des sondes (4 à 15 %) et normalisées à 100 % pour l'hybridation homologue (75 à 95 %). L'abréviation ND signifie : non déterminé. Les importances relatives des hybridations croisées entre les ADN-HPV dans les conditions sus-indiquées sont exprimées en % d'hybridation entre un ADN HPV marqué et un ADN HPV non marqué.

On constate l'absence ou la quasi-absence d'hybridation croisée entre les génomes des HPV1, 2, 4, 6b, 7 et 11a et d'ADNs d'HPV d'EV nouvellement clonés marqués au $^{32}$P ou entre les ADNs d'HPV d'EV non marqués et des sondes spécifiques des HPV13, 16 et 18. De façon semblable, on n'a pas ou quasiment pas détecté d'hybridation croisée entre les ADNs des HPV14a, 14b, 15, 17a, 17b, 19, 20, 21, 22, 23 et 24 et les ADNs des HPV1a et 11a par réassociation à saturation (tableau 2). Les ADNs d'HPV nouvellement

TABLEAU 2. DEGRE D'HYBRIDATION CROISEE ENTRE LES ADNs d'HPV DETERMINEE PAR HYBRIDATION EN PHASE LIQUIDE

| ADN d'HPV non marqué | % d'hybridation avec de l'ADN d'HPV marqué au $^{32}$P | | | | | | | | | | | | | |
|---|---|---|---|---|---|---|---|---|---|---|---|---|---|---|
| | 3a | 5 | 14a | 14b | 19 | 20 | 21 | 22 | 23 | 9 | 15 | 17a | 17b | 24 |
| 1a | 0,1 | 0,3 | 0,2 | 0,3 | 0 | 0,8 | 2,9 | 0 | 0 | 1,0 | 0,4 | 0,2 | 0 | 0 |
| 11a | 1,6 | 1,0 | 1,3 | 0 | 0,3 | 0,1 | 0,7 | 3,7 | 0 | 0,1 | 0,1 | 0,6 | 3,3 | 0 |
| 3a | 100 | 1,8 | 1,0 | 0 | 1,5 | 0,1 | 1,5 | 0 | 1,9 | 0,1 | 1,7 | 1,2 | 1,8 | 3,0 |
| 10 | 32,3 | ND | ND | 0,1 | 0 | 0,1 | 1,9 | 3,0 | ND | 0 | 1,6 | 0,1 | 2,0 | ND |
| 5 | 0,2 | 100 | 12,1 | 12,4 | 5,8 | 9,3 | 9,4 | 10,1 | 5,8 | 4,3 | 0,7 | 3,6 | 0 | 2,4 |
| 8 | 1,1 | 15,7 | 9,9 | 13,4 | 8,5 | 5,6 | 5,0 | 7,1 | 5,8 | 3,5 | 1,5 | 3,2 | 3,8 | 0,1 |
| 12 | 0,1 | 19,3 | 9,2 | 12,5 | 5,3 | 8,6 | 9,3 | 11,7 | 4,0 | 3,6 | 1,2 | 0,1 | 1,9 | 0 |
| 14a | 0,2 | 13,2 | 100 | 88,8 | 14,6 | 32,4 | 32,9 | 10,1 | 24,6 | 3,0 | 2,2 | 2,4 | 4,1 | ND |
| 14b | ND | 10,5 | 94,1 | 100 | 9,3 | 28,4 | 35,4 | 9,5 | 28,2 | 0 | 0 | 0 | 0 | 0 |
| 19 | ND | 7,2 | 21,4 | 20,6 | 100 | 7,6 | 8,8 | 15,5 | 27,7 | 0 | 0 | 0 | 2,2 | 1,0 |
| 20 | ND | 9,9 | 28,8 | 37,9 | 6,2 | 100 | 25,4 | 13,7 | 14,1 | 0 | 0 | 2,1 | 0 | 3,6 |
| 21 | ND | 10,5 | 38,7 | 40,5 | 6,4 | 37,5 | 100 | 9,8 | 18,6 | 0,1 | 0 | 2,5 | 0,3 | 0 |
| 22 | ND | 7,2 | 7,4 | ND | 17,3 | 7,2 | 10,8 | 100 | 17,9 | 0 | 0 | 0,1 | 0,1 | 0 |
| 23 | ND | ND | ND | ND | ND | ND | ND | 21,2 | 100 | 0 | 0,5 | 0 | 0 | 0,1 |
| 9 | 0,4 | 3,1 | 0,5 | 1,2 | 0 | 2,0 | 1,0 | 0 | 0 | 100 | 5,5 | 6,3 | 5,4 | 0 |
| 15 | 0,4 | 3,3 | 2,1 | 3,3 | 0 | 0,1 | 0,8 | 0 | 0 | 7,8 | 100 | 22,5 | 21,6 | 0 |
| 17a | 0,7 | 1,2 | 1,4 | 2,8 | 0 | 0,1 | 0,1 | 0,8 | 1,4 | 7,6 | 19,5 | 100 | 92,7 | 0 |
| 17b | ND | ND | ND | 1,4 | 0 | 0,3 | 3,4 | ND | ND | ND | ND | 86,3 | 100 | ND |
| 24 | ND | ND | 0,1 | 2,6 | ND | ND | ND | 0,8 | 0 | 0,2 | 0 | 1,1 | 1,1 | 100 |

8

clonés n'ont pas ou quasiment pas présenté d'hybridation croisée ou ont présenté une hybridation croisée inférieure à 50 % entre eux et avec les génomes des autres HPV associés à l'EV (HPV3a, 5, 8, 9, 10 et 12), à l'exception des HPV14a et 14b, d'une part, et des HPV17a et 17b, d'autre part, qui ont présenté une forte hybridation croisée. Ces observations justifient le classement des nouveaux virus en neuf nouveaux types (HPV14, 15, 17, 19, 20, 21, 22, 23 et 24) plus deux sous-types des types 14 (HPV14a et b) et 17 (HPV17a et b).

De même les différents HPVs ont été classés en groupes, sur la base de leurs homologies (ou absence d'homologies) de séquences dans des conditions strictes d'hybridation moléculaire. Ces groupes, désignés par les lettres A à H, sont répertoriés dans le tableau III qui suit. Ce tableau mentionne les maladies qui ont été diagnostiquées chez les porteurs de ces HPVs (isolés ou en combinaison entre eux) et les potentiels oncogènes qui leur ont été reconnus.

TABLEAU III

CLASSIFICATION DES HPV FAISANT L'OBJET DE LA DEMANDE DE BREVET, EN FONCTION DE LEUR DEGRE D'HOMOLOGIE DE SEQUENCE NUCLEOTIDIQUE DETERMINE PAR HYBRIDATION MOLECULAIRE DANS DES CONDITIONS STRICTES

| Groupe[1] | Types[2] d'HPV | Homologies au sein du groupe | Maladies associées | Potentiel oncogène | Mélange de sondes |
|---|---|---|---|---|---|
| A | 1 | | Myrcémies | très faible | 1 |
| B | 2 | | Verrues vulgaires | faible | 1 |
| C | 3,10,28*,29* | 14 à 38 % | Verrues planes, Verrues intermédiaires, Kératoses séniles, Maladie de Bowen | modéré ; un virus apparenté, associé à des néoplasies intraépithéliales et des cancers cutanés, en cours de caractérisation | 2 |
| D | 4 | | Verrues vulgaires | très faible | 1 |
| E | 5,8,12,14*,19,20*,21*,22*,23 | 4 à 38 % | Epidermodysplasie verruciforme, Kératoses séniles ou actiniques, Maladie de Bowen, Carcinomes cutanés | HPV5,8 et 14 associés aux carcinomes d'EV; un virus apparenté, associé à des néoplasies intraépithéliales et des cancers cutanés, en cours de caractérisation | 3,4,7 |
| F | 9,15*,17* | 6 à 23 % | Epidermodysplasie verruciforme | | 5 |
| G | 24* | | Epidermodysplasie verruciforme | | 6 |
| H | 13,31* | | Hyperplasie épithéliale focale orale, leucoplasies orales | | 8 |
| I | 32* | | Néoplasies intraépithéliales cutanées, Maladie de Bowen | | 7,9 |

1) Les génomes des types d'HPV appartenant à des groupes différents ne présentent généralement pas d'homologie de séquence décelable dans des conditions strictes d'hybridation moléculaire. Les génomes des types d'HPV appartenant au même groupe présentent une homologie de séquence inférieure à 50 %.

2) Les nouveaux types d'HPV sont indiqués par un astérisque.

Les ADNs des HPV5, 8, 12, 14, 19, 20, 21, 22 et 23 présentent entre eux des taux d'hybridation croisée (homologies de groupes) variant de 5 à 38 %, et ne présentent une hybridation croisée notable (4 à 13 %) qu'avec les ADNs des HPV5,, 8 et 12. Ces virus font donc partie d'un groupe d'HPV d'EV précédemment défini (9).

De même les ADNs des HPV9, 15 et 17 qui présentent entre eux une hybridation croisée d'environ 20 % et une hybridation croisée d'environ 6 % avec l'ADN d'HPV9, appartiennent également à un groupe d'HPV d'EV déjà décrits (9). Les HPVs de types 13 et 31 peuvent être considérés comme appartenant à un même groupe. Enfin les HPVs de types 1, 2, 4, 24 et 32 qui ne présentent presque pas d'homologie avec les génomes des autres HPV sont considérés comme formant les premiers membres d'autres groupes distincts entre eux et des groupes précédents.

L'invention concerne encore plus particulièrement des fragments d'ADN, issus des ADN-HPVs sus-décrits, et plus particulièrement ceux correspondant respectivement aux gènes E6-E7 ; E1 : L2 : L1 et à leurs régions intergéniques. Les positions et longueurs relatives de ces divers fragments, vis-à-vis des sites pris comme origines (figures 1 à 9) sont indiquées dans le tableau IV qui suit.

TABLEAU IV

LOCALISATION PUTATIVE DES PRINCIPAUX GENES ET DE LA REGION INTERGENIQUE SUR LES CARTES PHYSIQUES DES GENOMES D'HPV

| Type d'HPV | Coordonnées des extrêmités 5' et 3' des fragments correspondant | | | | à la région intergénique |
| | E6 - E7 | E1 | L2 | L1 | |
|---|---|---|---|---|---|
| 1 | 44 - 34,5 | 35 - 11 | 95 - 75,5 | 76,5 - 56 | 56 - 44,5 |
| 3 | 18,5 - 9 | 9,5 - 85,5 | 69,5 - 50 | 51 - 30,5 | 30,5 - 19 |
| 5 | 6,5 - 97 | 97,5 - 73,5 | 57,5 - 38 | 39 - 18,5 | 18,5 - 7 |
| 8 | 63 - 53,5 | 54 - 30 | 14 - 94,5 | 95,5 - 75 | 75 - 63,5 |
| 9 | 42 - 32,5 | 33 - 9 | 93 - 73,5 | 74,5 - 54 | 54 - 42,5 |
| 10a | 49 - 39,5 | 40 - 16 | 0 - 80,5 | 81,5 - 61 | 61 - 49,5 |
| 10b | 93 - 83,5 | 84 - 60 | 44 - 24,5 | 25,5 - 5 | 5 - 93,5 |
| 12 | 23,5 - 14 | 14,5 - 90,5 | 74,5 - 55 | 56 - 35,5 | 35,5 - 24 |
| 14 | 8,5 - 99 | 99,5 - 75,5 | 59,5 - 40 | 41 - 20,5 | 20,5 - 9 |
| 15 | 39,5 - 30 | 30,5 - 6,5 | 90,5 - 71 | 72 - 51,5 | 51,5 - 40 |
| 17 | 46 - 36,5 | 37 - 13 | 97 - 77,5 | 78,5 - 58 | 58 - 46,5 |
| 24 | 24,5 - 15 | 15,5 - 91,5 | 75,5 - 56 | 57 - 36,5 | 36,5 - 25 |
| 28 | 47,5 - 38 | 38,5 - 14,5 | 98,5 - 79 | 80 - 59,5 | 59,5 - 48 |
| 29 | 89,5 - 80 | 80,5 - 56,5 | 40,5 - 21 | 22 - 1,5 | 1,5 - 90 |
| 31 | 89 - 78,5 | 80 - 53,5 | 33,5 - 15,5 | 17,5 - 96,5 | 96,5 - 92,5 |

La localisation des gènes sur le génome du HPV1 a été déduite de la séquence nucléotidique de ce génome (Brevet O. Danos, M. Katinka et M. Yaniv). Les cartes physiques des génomes des HPV3, 5, 8,, 9,

EP 0 192 001 B1

10a, 12, 14, 15, 17 et 24 ont été alignées par rapport à la carte physique et à la carte génétique du HPV1, et celle du HPV31, par rapport à la carte physique et à la carte génétique du HPV6b (E. Schwarz et al, EMBO J., 1983, 2, 2341-2348), après analyse au microscope électronique de molécules hétéroduplexes formées dans des conditions strictes (Tm -29°C) ou moins strictes (Tm -40°C) d'hybridation. Les cartes physiques des HPV10b, 28 et 29 ont été alignées par rapport aux cartes physiques des HPV3a et 10a après juxtaposition des sites d'enzymes de restriction conservés.

Les valeurs des coordonnées portées dans le Tableau IV indiquent la position, sur les cartes physiques présentées dans les figs. 1-9, des extrémités 5' et 3' des segments des génomes homologues des gènes E6 et E7, E1, L2 et L1 et de la région intergénique par rapport au génome du HPV1a ou, dans le cas du HPV31, par rapport au génome du HPV6b.

La région intergénique (comportant des éléments de régulation) et les gènes adjacents E6 et E7 (correspondant vraisemblablement aux gènes de transformation majeurs exprimés dans les tumeurs) ne présentent pas d'homologie de séquence décelable par analyse au microscope électronique de molécules hétéroduplexes formées, dans des conditions non strictes d'hybridation, entre des génomes de types d'HPV appartenant à des groupes différents, ou formées, dans des conditions strictes d'hybridation, entre les génomes de la plupart des types d'HPV appartenant au même groupe. Le gène E1 (impliqué principalement dans la réplication de l'ADN viral) et le gène L1 (codant pour la protéine majeure de la capside virale portant les principaux déterminants antigéniques des virions) présentent des homologies de séquence décelables par analyse d'hétéroduplex formés, dans des conditions non strictes d'hybridation, entre des génomes de types d'HPV appartenant à des groupes différents ou formés, dans des conditions strictes d'hybridation, entre des génomes d'HPV appartenant au même groupe.

Des sondes préparées à partir de plasmides recombinants comportant les régions E1 et L1 peuvent théoriquement permettre de détecter le plus grand nombre de types d'HPV par des expériences d'hybridation moléculaire effectuées, selon le cas, dans des conditions strictes ou non strictes. Des sondes préparées à partir de plasmides recombinants comprenant la région intergénique et les gènes E6 et E7 permettent de détecter spécifiquement un type d'HPV ou des types d'HPV apparentés.

La région L2 (codant pour un constituant mineur de la capside virale) présente un degré variable de conservation de séquences nucléotidiques parmi les différents types d'HPV.

Dans ce qui suit sont encore décrites de façon plus précise les conditions dans lesquelles les virus HPV-IP2 et HPV-IP4 ont été isolés, puis les conditions dans lesquelles ont été obtenus les ADN-HPVs à partir de ces virus.

Clonage moléculaire et caractérisation d'un nouveau type d'HPV associé à des néoplasies et à des cancers génitaux (HPV IP2).

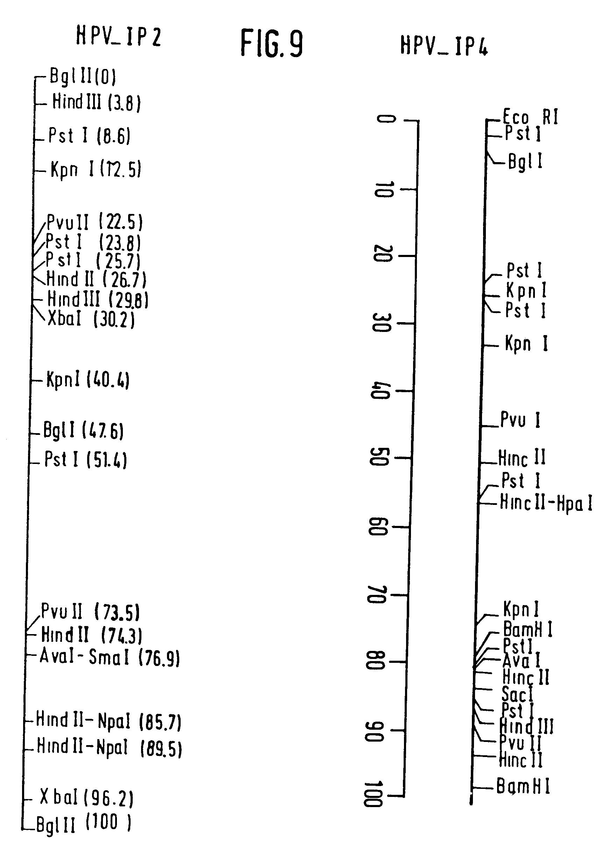

Un nouveau type d'HPV a été mis en évidence dans l'ADN extrait d'un cancer du col de l'utérus, par hybridation, dans des conditions non strictes, avec une sonde radioactive spécifique du HPV type 16. Aucune hybridation croisée n'était détectable lorsque l'hybridation était effectuée dans des conditions strictes d'hybridation. Une étude de la sensibilité de l'ADN de cet HPV à plusieurs enzymes de restriction a montré que l'enzyme BglII coupait une fois l'ADN viral. Après digestion de l'ADN extrait de la tumeur par l'endonucléase BglII, la fraction renfermant des molécules d'ADN de 8kb (taille d'un génome de papillomavirus) ont été purifiés par centrifugation dans un gradient de saccharose. Les molécules de 8kb ont été insérées, par le site BglII, dans un vecteur constitué du plasmide PL15.5 (renfermant un site unique de coupure par BglII et par BamHI) inséré par son site BamHI, dans l'ADN du bactériophage lambda L47.1. Après encapsidation de l'ADN recombinant et infection de bactéries hôtes (Escherichia coli, souche LA101), les plages de lyse correspondant à des phages recombinants ont été détectées par hybridation de répliques des cultures bactériennes infectées, avec un ADN de HPV16 radioactif, dans des conditions non strictes. Plusieurs bactériophages recombinants, contenant la totalité des séquences virales, ont été isolés : la coupure de l'ADN phagique par l'enzyme d'insertion BglII engendre un fragment de 8kb hybridant avec la sonde HPV16 dans des conditions non strictes ; la coupure de l'ADN des phages recombinants et de l'ADN de la tumeur d'origine par mélange des enzymes BglII et PstI engendre les mêmes 5 fragments dans la somme des poids moléculaires est égale à la taille d'un génome des papillomavirus. L'ADN du nouvel HPV a été excisé de l'ADN des bactériophages recombinants, purifié par électroélution, et recloné dans le plasmide PL15.5. Une carte de restriction de l'ADN viral a été établie à partir de la sensibilité de cet ADN à 18 endonucléases de restriction, ce qui a permis de localiser 21 sites de coupure (figure 9). La carte ainsi établie est différente de la carte des génomes des HPV identifiés à ce jour. L'homologie de séquence entre l'ADN du nouvel HPV et l'ADN des HPV identifiés à ce jour a été analysée par des expériences d'hybridation moléculaire sur réplique effectuées dans des conditions strictes. L'homologie détectée toujours été inférieure à 5 %, la plus grande homologie étant détectée avec le génome du HPV16. Le nouveau virus caractérisé à partir d'un cancer du col de l'utérus constitue donc un nouveau type d'HPV, provisoirement dénommé HPVIP2.

L'analyse, au microscope électronique, de molécules hétéroduplexes formées, dans différentes conditions, entre l'ADN de HPVIP2 et l'ADN du HPV1 a permis d'aligner les cartes physiques de ces 2 génomes et de définir la position théorique des différents gènes portés par l'ADN de l'HPVIP2.

LOCALISATION PUTATIVE DES PRINCIPAUX GENES ET DE LA REGION INTRAGENIQUE DU HPV-IP2 SUR LA CARTE DE CE GENOME

| | Coordonnées des extrémités | |
|---|---|---|
| | 5' | 3' |
| E6-E7 | 62 | 71,5 |
| E1 | 71 | 95 |
| E2 | 95,5 | 11,5 |
| L2 | 11 | 30,5 |
| L1 | 31,5 | 52 |
| Région intergénique | 52 | 63,5 |

L'utilisation de sondes radioactives préparées à partir de l'ADN de l'HPVIP2 purifié a permis de déterminer le pouvoir pathogène de ces virus. L'ADN du HPVIP2 a été mis en évidence dans un cas de papules bowenoïdes des organes génitaux externes sur les 14 étudiés, dans 2 cancers invasifs du col de l'utérus sur les 51 étudiés et dans 1 cas de néoplasie intraépithéliale du col de l'utérus sur les 28 étudiés. HPVIP2 constitue donc un type d'HPV à tropisme génital présentant un potentiel oncogène, dont la fréquence est un peu inférieure à celle du HPV18, et nettement inférieure à celle du HPV16. Il est nécessaire de l'incorporer à tout mélange d'ADN d'HPV destiné à la préparation de sondes moléculaires, en vue du diagnostic ou du dépistage des types d'HPV constituant un risque pour le développement de néoplasies génitales et, en particulier, de cancers du col de l'utérus.

Clonage moléculaire et caractérisation d'un nouveau type d'HPV associé à des lésions précancéreuses de la peau (HPV IP4).

Un nouveau type d'HPV a été mis en évidence dans l'ADN extrait d'une biopsie de kératose actinique, une lésion précancéreuse cutanée, par hybridation moléculaire, dans des conditions strictes, avec un mélange avec des sondes radioactives spécifiques des HPV type 5, 8 et 14. Aucune hybridation croisée n'a été détectée lorsque l'hybridation a été effectuée des sondes spécifiques des types 1,2,3,7,10,13,16,18,28,IP1 (précédemment dénommé HPV31), IP2, et IP3 (précédemment dénommé HPV32).

Une étude de la sensibilité de l'ADN de cet HPV a plusieurs enzymes de restriction a montré que l'enzyme EcoRI coupe une fois l'ADN viral. Après digestion de l'ADN extrait de la biopsie par l'endonucléase EcoRI, la fraction renfermant des molécules d'ADN de 8kb (taille d'un génome de papillomavirus) a été purifiée par centrifugation dans un gradient de saccharose. Les molécules 8kb ont été insérées, par le site EcoRI, dans l'ADN du bactériophage λgt wes. λB. Après encapsidation de l'ADN recombinant et infection de bactéries hôtes (Escherichia coli, souche LA101), les plages de lyse correspondant à des phages recombinants ont été détectées par hybridation de répliques des cultures bactériennes infectées, avec un mélange radioactif des ADN de HPVs, 8, et 14, dans des conditions non strictes. Plusieurs bactériophages recombinants, contenant la totalité des séquences virales ont été isolés : la coupure de l'ADN phagique par l'enzyme d'insertion EcoRI engendre un fragment de 8kb hybridant avec la sonde spécifique des HPV5,8 et 14 dans des conditions non strictes ; la coupure de l'ADN de phages recombinants et de l'ADN de la lésion d'origine par le mélange des enzymes EcoRI et PstI engendre les mêmes 6 fragments dont la somme des poids moléculaires est égale à la taille d'un génome des papillomavirus. L'ADN du nouvel HPV a été excisé de l'ADN d'un bactériophage recombinant, purifié par électroélution, et recloné dans le plasmide pSP65. Une carte de restriction de l'ADN viral a été établie à partir de la sensibilité de cet ADN à 15 endonucléases de restriction, ce qui a permis de localiser 23 sites de coupure (figure 10). La carte ainsi établie est différente de la carte des génomes des HPV identifiés à ce jour. L'homologie de séquence entre l'ADN du nouvel HPV et l'ADN des HPV identifiés à ce jour a été analysée par de expériences d'hybridation moléculaire sur réplique effectuées dans des conditions strictes. Une homologie, inférieure à 50 %, a été détectée entre l'ADN du nouvel HPV et l'ADN de certains types d'HPV précédemment identifiés dans des lésions d'épidermodysplasie verruciforme (HPV5,8,12,14,-19,20,21 et 25), mais aucune homologie n'a été détectée avec les autres types d'HPV. Le nouveau virus caractérisé à partir d'une kératose actinique constitue donc un nouveau type d'HPV provisoirement dénommé HPV-IP4.

L'utilisation d'une sonde radioactive préparée à partir de l'ADN de l'HPVIP4 purifié a permis de mettre en évidence HPVIP4 chez 42 % des 17 patients atteints d'épidermodysplasie verruciforme étudiés et dans x sur y des biopsies de kératose actinique analysées. Du fait de sa grande fréquence chez les malades atteints d'épidermodysplasie verruciforme, une maladie caractérisée par le développement fréquent de cancers cutanés, et de son association à une fraction des lésions de kératose actinique considérées comme précurseurs de cancers spinocellulaires de la peau, HPVIP4 constitue un type d'HPV à tropisme cutané présentant un potentiel oncogène. Il est nécessaire de l'incorporer à tout mélange d'ADN d'HPV destiné à la préparation de sondes moléculaires en vue du diagnostic ou du dépistage des types d'HPV constituant un risque pour le développement de lésions précancéreuses ou cancéreuses de la peau.

L'invention concerne plus particulièrement encore des mélanges ou cocktails de différents ADNs HPVs (ou sondes contenant ces ADNs HPVs ou des séquences de ces derniers), susceptibles d'être utilisés en combinaison pour réaliser des diagnostics globaux des différentes formes d'infections à papillomavirus, éventuellement aux fins de pronostiquer l'évolution possible de l'infection. Des mélanges préférés conformes à l'invention sont identifiés dans le tableau V qui suit.

TABLEAU V

CARACTERISTIQUES DES MELANGES D'ADN D'HPV UTILISABLES POUR LE DIAGNOSTIC VIROLOGIQUE D'INFECTIONS A PAPILLOMAVIRUS

| Désignation des mélanges | Constitution [1] | Maladies à diagnostiquer |
|---|---|---|
| 1 | 1,2d*,4 | Verrues cutanées ou muqueuses (en particulier, verrues vulgaires et plantaires). Diagnostic différentiel de l'épidermodysplasie verruciforme. |
| 2 | 3,10a,10b*,28*,29* | Verrues planes ou intermédiaires cutanées ou muqueuses. Néoplasies intraépithéliales et cancers cutanés. Diagnostic différentiel de l'épidermodysplasie verruciforme. |
| 3 | 5,17a*,24* | Epidermodysplasie verruciforme. Néoplasies intraépithéliales et cancers cutanés. |
| 4 | 5,8,12,14a*,14b,19*,20*,21*,22*,23* | Epidermodysplasie verruciforme. |
| 5 | 9,15*,17a,17b | Epidermodysplasie verruciforme. |
| 6 | 24* | Epidermodysplasie verruciforme. |
| 7 | 5,8,14b*,32* | Cancers cutanés de l'épidermodysplasie verruciforme. Néoplasies intraépithéliales et cancers cutanés. |
| 8 | 13,31* | Hyperplasie épithéliale focale orale ; Diagnostic différentiel des néoplasies intraépithéliales orales. |
| 9 | 32* | Néoplasies intraépithéliales et cancers cutanés. |

1) Les nouveaux types d'HPV entrant dans la constitution des mélanges de sondes sont indiqués par un astérisque.

Ce tableau indique également les natures des affections susceptibles d'être plus particulièrement diagnostiquées par l'utilisation des mélanges figurant dans la partie gauche du tableau. Il sera remarqué que les regroupements des cartes de restriction dans les figures 1 à 9 ci-jointes sont en conformité avec les regroupements qui sont indiqués dans la colonne "Constitution" du tableau V. C'est également la raison pour laquelle certaines des sondes ont été reproduites à plusieurs reprises dans différentes figures des dessins ci-annexés.

L'invention concerne encore un nécessaire ou kit comportant une pluralité de sondes ou mélanges de sondes distinctes, caractérisé par neuf groupes de sondes ; chacun desquels comporte :

1) au moins l'ADN cloné ou recombinant de l'HPV 2d (C.N.C.M. n° I-379) ;

2) au moins un ADN choisi parmi les ADNs clonés ou recombinants de HPV 10b (C.N.C.M. n° I-380 et n° I-381), HPV 28 (C.N.C.M. n° I-394) et HPV 29 (C.N.C.M. n° I-395);

3) au moins un ADN choisi parmi les ADNs clonés ou recombinants de HPV 17 (C.N.C.M. n° I-385 et n° I-386), et HPV 24 (C.N.C.M. n° I-392 et n° I-393) ;

4) au moins un ADN choisi parmi les ADNs clonés ou recombinants de HPV 14 (C.N.C.M. n° I-382 et n° I-383), HPV 15 (C.N.C.M. n° I-384), HPV 17 (C.N.C.M. n° I-385 et n° I-386), HPV 19 (C.N.C.M. n° I-387), HPV 20 (C.N.C.M. n° I-388), HPV 21 (C.N.C.M. n° I-389), HPV 22 (C.N.C.M. n° I-390) et HPV 23 (C.N.C.M. n° I-391) ;

5) au moins un ADN choisi parmi les ADNs clonés ou recombinants de HPV 15 (C.N.C.M. n° I-384) et HPV 17 (C.N.C.M. n° I-385 et n° I-386) ;

6) l'ADN cloné ou recombinant de HPV 24 (C.N.C.M. n° I-392 et n° I-393) ;

7) au moins un ADN choisi parmi les ADNs clonés ou recombinants de HPV 14 (C.N.C.M. n° I-382 et n° I-383), et HPV 32 (C.N.C.M. n° I-397) ;

8) l'ADN cloné ou recombinant de HPV 31 (C.N.C.M. n° I-396) ;

9) l'ADN cloné ou recombinant de HPV 32 (C.N.C.M. n° I-397) ;

étant entendu que chacun de ces ADNs recombinants ou clonés peut être remplacé par un ADN cloné correspondant ou fragment d'ADN qui hybride avec le précédent dans des conditions strictes : dans les conditions décrites par KREMSDORF D. et al. (1983), J. Virol. 48: 340-351 :

chauffage de ces ADNs recombinants, après dénaturation et transfert préalable sur des filtres de transfert pour hybridation, à 80°C pendant 4 heures, préhybridation pendant la nuit à 42°C des filtres dans une solution de Denhardt, 2 x SSC, phosphate de sodium 50 mM (pH 6,5), ADN de thymus de veau (250 $\mu$g/ml) et formamide déionisé 35%, puis hybridation dans la même solution pendant 48 heures à 42°C avec l'ADN viral marqué par $^{32}$P (2 x 10$^4$ cpm/cm$^2$) et lavage des filtres sous agitation pendant 30 minutes à 50°C dans une solution de Trischlorhydrate 60 mM (pH8), EDTA 2 mM, NaCl 300 mM et dodécyl-sulfate de sodium, puis pendant 30 minutes à température ambiante dans une solution Tris 3 mM

que les ADNs ou fragments d'ADN des neuf groupes sont choisis de façon à être en toutes circonstances différents les uns des autres, dans la mesure où chacun des neuf groupes serait réduit à un seul des ADNs qui le composent.

Etant donné la grande diversité des HPVs susceptibles d'être isolés à partir des différentes formes de verrues ou autres lésions cutanées ou de muqueuses, il est cependant préféré d'utiliser, pour le diagnostic de chaque type d'affection mentionné dans le tableau, des mélanges comportant plus d'un ou deux ADN-HPVs, dès lors que d'autres ADN-HPVs ont été reconnus comme pouvant également intervenir dans le développement du même type d'affection. Le diagnostic de la nature de l'infection et de son évolution possible sera d'autant plus efficace que le nombre de sondes utilisées sera plus élevé. En outre, des essais d'hybridation réalisés avec des mélanges différents de sondes permettra des diagnostics différentiels présentant un degré de probabilité également plus important de la nature du mal dont souffre le patient.

Dans le tableau V, il n'a été mentionné que des sondes formées à partir d'ADN-HPVs isolés dans le laboratoire des inventeurs. Il va sans dire que, en raison de ce qui précède, les différents mélanges pourront avantageusement être complétés avec des ADNs issus des HPVs obtenus dans d'autres laboratoires, dès lors qu'ils auront été retrouvés à différentes reprises chez des patients affectés par les mêmes types d'infections. Par exemple, le mélange 7 ne peut que gagner à être complété par tous autres ADN-HPVSs rencontrés dans des épidermodysplasies verruciformes à risque de transformation en néoplasies intraépithéliales et cancers cutanés. On remarquera que dans le tableau V, certains des mélanges sont présentés comme caractéristiques des mêmes maladies à diagnostiquer. Il est à remarquer cependant que les différents mélanges font une distinction entre des infections à faible risque de cancérisation et des infections à haut risque de cancérisation. Par exemple, l'hybridation d'une préparation virale provenant d'un malade soumis à diagnostic avec le mélange 7 témoignera d'un risque de cancérisation cutanée plus important que dans le cas où l'hybridation se produira plutôt avec le mélange 3.

De même les EV détectees par le mélange 5 temoigneront d'un risque de cancérisation plus important que les EV détectées par le mélange 6. Le mélange 4 détectera des EV à risque plus élevé encore que celles détectées par le mélange 5.

On décrit encore ci-après d'autres mélanges ou cocktails de différents ADNs-HPVs (ou sondes contenant ces ADNs-HPVs ou de séquences de ces derniers), susceptibles d'être utilisés en combinaison pour réaliser des diagnostics globaux des différentes formes d'infections à papillomavirus, éventuellement aux fins de pronostiquer l'évolution possible de l'infection.

Des mélanges préférés conformes à l'invention sont identifiés dans le tableau V susdit.

Le tableau susdit indique également les natures des affections susceptibles d'être plus particulièrement diagnostiquées par l'utilisation des mélanges figurant dans la partie gauche du tableau. Il est rappelé que les cartes de restriction des autres ADN-HPVs identifiés dans le tableau qui précède sont contenues dans les figures 1 à 9.

Il est à remarquer que le HPV-IP2 peut être considéré comme particulièrement représentatif de sondes utilisables pour la détection des risques de développement de néoplasies génitales et, en particulier, de cancers du col de l'utérus.

L'invention concerne donc plus particulièrement encore des trousses ou "kits" de diagnostic comprenant au moins 10 groupes figurant dans les groupes numérotés de 1 à 10 dans le tableau sous la rubrique "Désignation des mélanges".

Dans ce qui précède, on a surtout envisagé l'utilisation, à titre de sondes, des ADN-HPVs entiers clonés. Ceux-ci peuvent cependant être substitués par des fragments clonés de ces différents ADNs, notamment par les gènes E1 ou L1 et par les gènes E6-E7.

Le principe de base des détections in vitro d'ADN-HPV mettra naturellement en jeu des hybridations opérées dans des conditions strictes ou moins strictes. On peut opérer par exemple comme suit, étant naturellement entendu que les essais de diagnostic décrits ne sauraient être considérés comme limitatifs des conditions d'emploi des sondes ou mélanges de sondes selon l'invention.

L'objet des examens mettant en jeu des sondes préparées à partir de mélanges d'ADNs de HPVs clonés est de mettre en évidence un HPV et d'identifier le type d'HPV dans une biopsie, dans des cellules obtenues par grattage de lésions, ou dans des coupes de biopsies fixées par le mélange de Carnoy (éthanol, chloroforme, acide acétique 6:3:1) et incluses dans la paraffine. L'examen nécessite l'extraction préalable de l'ADN des prélèvements selon des méthodes dont le principe est connu et met en jeu l'analyse de cet ADN par des expériences d'hybridation moléculaire, effectuées dans des conditions strictes ou moins strictes, à l'aide de sondes radioactives (marquées au $^{32}$P ou au $^{35}$S) préparées à partir de mélanges d'ADNs d'HPVs. Chaque examen requiert, en général, l'utilisation de plusieurs mélanges de sondes.

Plusieurs méthodes d'hybridation peuvent être utilisées. On peut, par exemple, mettre en oeuvre la méthode d'hybridation sur tache. Cette méthode comporte, après dénaturation de l'ADN, le dépôt d'une quantité aliquote d'ADN sur des membranes (nitrocellulose ou Genescreenplus), l'hybridation de chaque membrane, dans les conditions usuelles, avec un mélange de sondes et la détection des hybrides radioactifs par exposition des membranes au contact d'un film radiographique. On peut aussi utiliser une méthode d'hybridation sur réplique. Cette méthode comprend la séparation électrophorétique en gel d'agarose des fragments d'ADN engendrés après traitement de l'ADN par des enzymes de restriction, le transfert des fragments, après dénaturation alcaline, sur des membranes (nitrocellulose, Genescreenplus) et leur hybridation, dans les conditions usuelles, avec différents mélanges de sondes. La formation d'hybrides radioactifs est détectée après exposition des membranes au contact d'un film radiographique.

Les sondes radioactives sont constituées soit par des ADNs d'HPVs marqués par la méthode de "nick-translation", soit par des ARNs préparés par transcription d'ADNs viraux insérés dans un vecteur, par exemple de type SP6. L'utilisation de sondes radioactives présente l'avantage d'une grande sensibilité, mais ceci n'exclut pas l'utilisation de sondes non radioactives par exemple biotinylées et susceptibles d'être reconnues par des anticorps soit marqués eux-mêmes, soit eux-mêmes reconnus par des anticorps portant un marqueur enzymatique, fluorescent, etc..

Le choix des sondes dépend de la nature des prélèvements. Ainsi, par exemple, dans le cas d'un malade suspect d'être atteint de EV, les mélanges 1, 2, 3, 4, 5, 6 et 7 seront utilisés. Les mélanges 1 et 2 permettront de faire le diagnostic différentiel entre l'EV et les verrues cutanées. La sonde 3, incluant le membre le plus fréquemment détecté de chacun des trois groupes d'HPVs associés à la maladie, et la sonde 7, contenant les ADNs des types d'HPV associés aux cancers d'EV, permettront le diagnostic de la majorité des cas d'EV et, en particulier, d'identifier les patients infectés par les types d'HPVs présentant un risque pour le développement de cancers. L'utilisation des mélanges 4, 5 et 6 permettront de préciser le type ou les types d'HPV infectant un même malade.

L'invention concerne donc encore des nécessaires ou "kits" contenant une pluralité des sondes sus-indiquées, notamment :

- soit des représentants de chacun des 19 types et sous-types d'ADN-HPVs sus-indiqués,
- soit des mélanges de sondes, de préférence les divers groupes ou mélanges de sondes qui ont été définis plus haut,

ces "kits" étant destinés à des études de diagnostic "in vitro par hybridation entre des préparations virales obtenues à partir de patients et les divers groupes ou mélanges.

Comme il va de soi et comme il résulte d'ailleurs déjà de ce qui précède, l'invention ne se limite nullement à ceux de ses modes d'application et de réalisation qui ont été plus spécialement envisagés ; elle en embrasse au contraire toutes les variantes ; notamment la référence dans les revendications à une désignation ADN-HPV suivie d'un nombre déterminé, et à laquelle correspond un ADN-HPV dont la carte de restriction a été fournie dans les dessins, s'entend comme signifiant que ces revendications couvrent tous les ADN-HPVs qui ont en commun avec cet ADN-HPV particulier de pouvoir être classés dans le même type, selon la définition qui en a été donnée plus haut, et a fortiori aux ADN-HPV appartenant au même sous-type.

Il est encore remarqué, en ce qui concerne plus particulièrement l'ADN dérivé de HPV-32, qui apparaît dans les, dessins, n'est pas coupé par AvaI, BalI, BamHI, ClaI, EcoRI, HindIII, NdeI, NruI, PvuI, PvuII, SacI, SalI, SmaI, TthIII, XmaI.

On notera que les ADNs recombinants désignés ci-après ont été déposés le 30 novembre 1984 à la C.N.C.M. (Collection Nationale des Cultures de Micro-Organismes de l'INSTITUT PASTEUR de Paris), sous les numéros apparaissant ci-après :

| | |
|---|---|
| pBR322/HPV2d | n° I-379 |
| pBR322/HPV10bA | n° I-380 |
| pBR322/HPV10bB | n° I-381 |
| pBR322/HPV14a | n° I-382 |
| pBR322/HPV14b | n° I-383 |
| pBR322/HPV15 | n° I-384 |
| pBR322/HPV17a | n° I-385 |
| pHPV5 HindIIIB/HPV17b | n° I-386 |
| pBR322/HPV19 | n° I-387 |
| pBR322/HPV20 | n° I-388 |
| pBR322/HPV21 | n° I-389 |
| pHV5 HindIIIB/HPV22 | n° I-390 |
| pBR322/HPV23 | n° I-391 |
| pBR322/HPV24a | n° I-392 |
| pBR322/HPV24b | n° I-393 |
| pBR322/HPV28 | n° I-394 |
| pBR322/HPV29 | n° I-395 |
| pBR322/HPV31 | n° I-396 |
| pSP64/HPV32 | n° I-397 |
| pLI55/IP2 | n° I-450 |
| pSP65/IP4 | n° I-449 |

L'invention concerne encore plus particulièrement les produits d'expression des gènes E6 et E7 des différents papillomavirus qui ont été évoqués dans ce qui précède et qui peuvent être utilisés comme principes actifs de vaccins susceptibles d'induire, lorsqu'ils sont administrés à des doses efficaces, la résistance de l'hôte au développement de néoplasies associées aux papillomavirus.

L'invention concerne également les sérums susceptibles d'être obtenus par immunisation d'un mammifère, sérums qui peuvent être utilisés pour la préparation de sérums administrables en doses efficaces à un patient, notamment par voie parentérale, ces sérums étant alors susceptibles de provoquer une régression des infections induites par les papillomavirus de types ou sous-types correspondants.

Il n'est point besoin d'insister sur la capacité de l'homme du métier d'obtenir les produits d'expression polypeptides du genre en question, notamment par des techniques du génie génétique consistant à incorporer les séquences E6 et/ou E7 dans un vecteur sous le contrôle d'un promoteur approprié, puis à transformer un hôte cellulaire, dont les papillomavirus sont susceptibles de reconnaître les promoteurs en question et d'exprimer les séquences qui lui sont associées.

L'invention concerne donc encore les compositions à usage pharmaceutique contenant les principes du genre en question (produits d'expression ou anticorps correspondants), en association avec un véhicule pharmaceutique physiologiquement acceptable. En particulier, ce dernier est constitué par une solution saturée injectable, dans le cas où les compositions du genre en question seront à administrer par voie parentérale.

Il est enfin fait référence aux articles dont les références bibliographiques suivent, lesquels complètent en tant que de besoin la description de l'état de la technique antérieur, dans la mesure où cela pourrait s'avérer utile à la compréhension complète du texte par le lecteur. A ce titre, le contenu de ces articles doit donc être considéré comme faisant partie de la description.

BIBLIOGRAPHIE

(1) Dèrst, M. et al., 1983, Proc. Natl. Acad. Sci. U.S.A., 80:3812-3815.
(2) Coggin, J.R., Jr. et al., 1979, Cancer Res., 39:545-546.
(3) Gissmann, L. et al., 1982, J. Virol. 44:393-400.
(4) Green, M. et al., 1982, Proc. Natl. Acad. Sci. U.S.A. 79:4437-4441.
(5) Heilman, C.A. et al., 1980, Virol. 36:395-407.
(6) Jablonska, S. et al., 1972, Cancer Res., 32:583-589.
(7) Jablonska, S. et al., 1982, Springer Semin. Immunopathol. 5:33-62.
(8) Kremsdorf, D. et al., 1982, J. Virol. 43:436-447.
(9) Kremsdorf, D. et al., 1983, J. Virol. 48:340-351.
(10) Lutzner, M.A. et al., 1978, Bull. Cancer, 65:169-182.
(11) Lutzner, M.A. et al., 1983, Lancet ii:422-424.
(12) Migozzi, M. et al., 1965, Bull. Soc. Franc. Derm. Syph. 72:747-748.
(13) Orth, G. et al., 1980, Cold Spring Harbor Conf. Cell Proliferation, 7:259-282.
(14) Orth, G. et al., 1981, J. Invest. Dermatol. 76:97-102.
(15) Orth, G. et al., 1979, Cancer Res. 39: 1074-1082.
(16) Ostrow, R.S. et al, 1982, Proc. Natl. Acad. Sci. U.S.A., 79:1634-1638.
(17) Ostrow, R.S. et al., 1983, Ann. Acad. Dermatol. 8:398-404.
(18) Pfister, H. et al., 1983, Cancer Res. 43:1436-1441.
(19) Pfister, H. et al., 1983, J Virol. 47:363-366.
(20) Pfister, H. et al., 1981, Int. J Cancer, 27:645-650.
(21) Rueda, L.A. et al., 1976, Med. Cut. I.L.A. 2:113-136.
(22) Ruiter, M. et al. J Invest. Dermatol., 47:247-252.
(23) Sutcliffe, J.G., 1978, Nucleic Acids Res. 5:2721-2728.
(24) Tsumori, T. et al., 1983, J. Gen. Virol. 64:967-969.

**Revendications**

1. Composition utilisable pour la détection de papillomavirus dans un milieu biologique le contenant, caractérisée en ce qu'elle contient un ou plusieurs ADN-HPVs distincts clonés choisis parmi les ADNs clonés déposés le 30 novembre 1984 à la C.N.C.M. (Collection Nationale de Cultures de Microorganismes de l'INSTITUT PASTEUR de Paris, sous les numéros ci-après :

```
HPV 2d ................. n° I-379

HPV 10bA ............... n° I-380

HPV 10bB ............... n° I-381

HPV 28 ................. n° I-394

HPV 29 ................. n° I-395

HPV 31 ................. n° I-396

HPV 32 ................. n° I-397

IP-2 ................... n° I-450

IP-4 ................... n° I-449
```

- ou un ou plusieurs ADNs clonés correspondants qui hybrident avec l'un de ceux qui ont été déposés auprès de la CNCM, dans des conditions strictes : chauffage de ces ADNs recombinants, après dénaturation et transfert préalable sur des filtres de transfert pour hybridation à 80°C pendant 4 heures, préhybridation pendant la nuit à 42°C des filtres dans une solution de Denhardt, 2 x SSC, phosphate de sodium 50 mM (pH 6,5), ADN de thymus de veau (250 $\mu$g/ml) et formamide déionisé 35%, puis hybridation dans la même solution pendant 48 heures à 42°C avec l'ADN viral marqué par $^{32}$P (2 x 10$^4$ cpm/cm$^2$) et lavage des filtres sous agitation pendant 30 minutes à 50°C dans une solution de Tris-chlorhydrate 60 mM (pH8), EDTA 2 mM, NaCl 300 mM et dodécyl sulfate de sodium 0,5%, puis pendant 30 minutes à température ambiante dans une solution Tris 3 mM

- ou un ou plusieurs fragments clonés choisis parmi les fragments issus des HPV-ADNs clonés déposés à la C.N.C.M. sous les numéros sus-indiqués et qui correspondent aux régions E1 ; E6 ; E7 ; L1 ; L2 ; ou à des régions intergéniques desdits ADN clonés

- ou un ou plusieurs fragments d'ADN clonés correspondants qui hybrident avec l'un des précédents, dans les susdites conditions strictes.

2. Nécessaire ou kit comportant une pluralité de sondes ou mélanges de sondes distinctes, caractérisé par neuf groupes de sondes ; chacun desquels comporte :

(1) au moins l'ADN cloné ou recombinant de l'HPV 2d (C.N.C.M. n° I-379) ;

(2) au moins un ADN choisi parmi les ADNs clonés ou recombinants de HPV 10b (C.N.C.M. N° I-380 et n° I-381), HPV 28 (C.N.C.M. n° I-394) et HPV 29 (C.N.C.M. n° I-395) ;

(3) au moins un ADN choisi parmi les ADNs clonés ou recombinants de HPV 17 (C.N.C.M. n° I-385 et n° I-386), et HPV 24 (C.N.C.M. n° I-392 et n° I-393) ;

(4) au moins un ADN choisi parmi les ADNs clonés ou recombinants de HPV 14 (C.N.C.M. N° I-382 et n° I-383), HPV 15 (C.N.C.M. n° I-384), HPV 17 (C.N.C.M. n° I-385 et n° I-386), HPV 19 (C.N.C.M. n° I-387), HPV 20 (C.N.C.M. n° I-388), HPV 21 (C.N.C.M. n° I-389), HPV 22 (C.N.C.M. n° I-390) et HPV 23 (C.N.C.M. n° I-391) ;

(5) au moins un ADN choisi parmi les ADNs clonés ou recombinants de HPV 15 (C.N.C.M. n° I-384) et HPV 17 (C.N.C.M. N° I-385 et n° I-386) ;

(6) l'ADN cloné ou recombinant de HPV 24 (C.N.C.M. n° I-392 et n° I-393) ;

(7) au moins un ADN choisi parmi les ADNs clonés ou recombinants de HPV 14 (C.N.C.M. N° I-382 et n° I-383), et HPV 32 (C.N.C.M. n° I-397) ;

(8) l'ADN cloné ou recombinant de HPV 31 (C.N.C.M. n° I-396) ;

(9) l'ADN cloné ou recombinant de HPV 32 (C.N.C.M. n° I-397) ;

étant entendu

- que chacun de ces ADNs recombinants ou clonés peut être remplacé par un ADN cloné correspondant ou fragment d'ADN qui hybride avec le précédent dans des conditions strictes : chauffage de ces ADNs recombinants après dénaturation et transfert préalable sur des filtres de transfert pour hybridation à 80°C pendant 4 heures, préhybridation pendant la nuit à 42°C des filtres dans une solution de Denhardt, 2 x SSC, phosphate de sodium 50 mM (pH 6,5), ADN de thymus de veau (250 $\mu$g/ml) et formamide déionisé 35%, puis hybridation dans la même solution pendant 48 heures à 42°C avec l'ADN viral marqué par $^{32}$P (2 x 10$^4$ cpm/cm$^2$) et lavage des filtres sous agitation pendant 30 minutes à 50°C dans une solution de Tris-chlorhydrate 60 mM (pH8), EDTA 2 mM, NaCl 300 mM et dodécyl sulfate de sodium, puis pendant 30 minutes à température ambiante dans une solution Tris 3 mM,

- que les ADNs ou fragments d'ADN des neuf groupes sont choisis de façon à être en toutes circonstances différents les uns des autres, dans la mesure où chacun des neuf groupes serait réduit à un seul des ADNs qui le composent.

3. Nécessaire ou kit selon la revendication 2, caractérisé en ce que les groupes comportent respectivement :

(1) un mélange d'ADNs clonés ou recombinants de HPV 1, 2d, 4 ou un mélange de fragments de ces ADNs ;

(2) un mélange d'ADNs clonés ou recombinants de ADN-HPV 3, 10a, 10b, 28 et 29 ou un mélange de fragments de ces ADNs ;

(3) un mélange d'ADNs clonés ou recombinants de ADN-HPV 5, 17a et 24 ou un mélange de fragments de ces ADNs ;

(4) un mélange d'ADNs clonés ou recombinants de ADN-HPV 5, 8, 12, 14a, 14b, 19, 20, 21, 22, et 23 ou un mélange de fragments de ces ADNs ;

(5) un mélange d'ADNs clonés ou recombinants de ADN-HPVs 9, 15, 17a et 17b ou un mélange de fragments de ces ADNS ;

(6) un ADN cloné ou recombinant de ADN-HPV 24 ou un mélange de fragments de ces ADNs ;

(7) un mélange d'ADNs clonés ou recombinants de ADN-HPVs 5, 8, 14b et 32 ou un mélange de fragments de ces ADNs ;

(8) un mélange d'ADNs clonés ou recombinants de ADN-HPVs 18 et 31 ou un mélange de fragments de ces ADNs ;

(9) un ADN cloné ou recombinant de ADN-HPV 32.

4. Nécessaire ou kit selon la revendication 2 ou la revendication 3, caractérisé en ce que le groupe 7) comporte en outre l'ADN cloné ou recombinant de HPV IP4 (C.N.C.M. N° I-449) ou un fragment cloné ou recombinant de cet ADN et en ce que ledit nécessaire ou kit comporte un dixième groupe comportant un mélange d'ADNs clonés ou recombinants de HPV 16, HPV 18 et IP 2 (C.N.C.M. n° I-450), ou un mélange de fragments de ces ADNs.

5. Nécessaire ou kit selon la revendication 4, caractérisé en ce que les fragments mis en oeuvre dans les groupes ou mélanges correspondent aux régions E1 ; E6 ; E7 ; L1 ; L2 ; ou à des régions intergéniques desdits fragments d'ADN-HPV.

6. Nécessaire ou kit selon l'une quelconque des revendications 2 ou 3, destiné aux diagnostics in vitro respectifs :

(1) des verrues cutanées ou muqueuses (en particulier, verrues vulgaires et plantaires) ; diagnostic différentiel de l'épidermodysplasie verruciforme ;

(2) des verrues planes ou intermédiaires cutanées ou muqueuses ; des néoplasies intraépithéliales et cancers cutanés ; diagnostic différentiel de l'épidermodysplasie verruciforme ;

(3) de l'épidermodysplasie verruciforme ; des néoplasies intra-épithéliales et cancers cutanés ;

(4) de l'épidermodysplasie verruciforme ;

(5) de l'épidermodysplasie verruciforme ;

(6) de l'épidermodysplasie verruciforme ;

(7) des cancers cutanés de l'épidermodysplasie verruciforme ; des néoplasies intraépithéliales et cancers cutanés ;

(8) de l'hyperplasie épithéliale focale orale ; du diagnostic différentiel des néoplasies intraépithéliales orales ;

(9) des néoplasies intraépithéliales et cancers cutanés.

7. Nécessaire ou kit selon la revendication 4 ou la revendication 5, destiné aux diagnostics in vitro respectifs :

(1) des verrues cutanées ou muqueuses (en particulier, verrues vulgaires et plantaires) ; diagnostic différentiel de l'épidermodysplasie verruciforme ;

(2) des verrues planes ou intermédiaires cutanées ou muqueuses ; des néoplasies intraépithéliales et cancers cutanés ; diagnostic différentiel de l'épidermodysplasie verruciforme ;

(3) de l'épidermodysplasie verruciforme ; des néoplasies intra-épithéliales et cancers cutanés ;

(4) de l'épidermodysplasie verruciforme ;

(5) de l'épidermodysplasie verruciforme ;

(6) de l'épidermodysplasie verruciforme ;

(7) des cancers cutanés de l'épidermodysplasie verruciforme ; des néoplasies intraépithéliales et cancers cutanés ;

(8) de l'hyperplasie épithéliale focale orale ; du diagnostic différentiel des néoplasies intraépithéliales orales ;

(9) des néoplasies intraépithéliales et cancers cutanés ;

(10) des néoplasies génitales et, en particulier, des cancers de l'utérus.

8. Procédé de détection et d'identification in vitro de papillomavirus contenus dans un échantillon biologique comprenant la réalisation d'essais d'hybridation distincts avec les ADN-HPVs clonés ou recombinants ou les fragments d'ADN-HPVs de ces nécessaires ou kits, selon l'une quelconque des revendications 2 à 7 et la détection de ceux des ADN-HPVs ou fragments clonés qui donnent lieu à une hybridation préférentielle avec les ADN-HPVs contenus dans l'échantillon biologique.

9. Procédé selon la revendication 8, caractérisé en ce que les essais d'hybridation sont faits avec les ADNs clonés entiers ou des fragments d'ADN correspondant aux régions E6 ou E7 dans les conditions d'hybridation stricte.

10. ADN cloné ou recombinant de HPV 2d (C.N.C.M. n° I-379) ou ADN cloné correspondant de cet ADN cloné qui hybride avec le précédent dans des conditions strictes : chauffage de ces ADNs recombinants, après dénaturation et transfert préalable sur des filtres de transfert pour hybridation, à 80°C pendant 4 heures, préhybridation pendant la nuit à 42°C des filtres dans une solution de Denhardt, 2 x SSC, phosphate de sodium 50 mM (pH, 6,5), ADN de thymus de veau (250 $\mu$g/ml) et formamide déionisé 35%, puis hybridation dans la même solution pendant 48 heures à 42°C avec l'ADN viral marqué par $^{32}$P (2 x 10$^4$ cpm/cm$^2$) et lavage des filtres sous agitation pendant 30 minutes à 50°C dans une solution de Tris-chlorhydrate 60 mM (pH8), EDTA 2 mM, NaCl 300 mM et dodécyl-sulfate de sodium 0,5%, puis pendant 30 minutes à température ambiante dans une solution Tris 3 mM
ou fragments d'ADN correspondant à l'une des régions E1 ; E6 ; E7 ; L1 ; L2 ou à une région intergénique de cet ADN-HPV ou fragment d'ADN correspondant hybridant avec le précédent dans les susdites conditions strictes.

**11.** ADN cloné ou recombinant de HPV 10b (C.N.C.M. n° I-380 et n° I-381) ou ADN cloné correspondant de cet ADN cloné qui hybride avec le précédent dans des conditions strictes : chauffage de ces ADNs recombinants, après dénaturation et transfert préalable sur des filtres de transfert pour hybridation, à 80°C pendant 4 heures, préhybridation pendant la nuit à 42°C des filtres dans une solution de Denhardt, 2 x SSC, phosphate de sodium 50 mM (pH 6,5), ADN de thymus de veau (250 $\mu$g/ml) et formamide déionisé 35%, puis hybridation dans la même solution pendant 48 heures à 42°C avec l'ADN viral marqué par $^{32}$P (2 x 10$^4$ cpm/cm$^2$) et lavage des filtres sous agitation pendant 30 minutes à 50°C dans une solution de Tris-chlorhydrate 60 mM (pH8), EDTA 2 mM, NaCl 300 mM et dodécyl-sulfate de sodium 0,5%, puis pendant 30 minutes à température ambiante dans une solution Tris 3 mM
ou fragments d'ADN correspondant à l'une des régions E1 ; E6 ; E7 ; L1 ; L2 ou à une région intergénique de cet ADN-HPV ou fragment d'ADN correspondant hybridant avec le précédant dans les susdites conditions strictes.

**12.** ADN cloné ou recombinant de HPV 28 (C.N.C.M. n° I-394) ou ADN cloné correspondant de cet ADN cloné qui hybride avec le précédent dans des conditions strictes : chauffage de ces ADNs recombinants, après dénaturation et transfert préalable sur des filtres de transfert pour hybridation, à 80°C pendant 4 heures, préhybridation pendant la nuit à 42°C des filtres dans une solution de Denhardt, 2 x SSC, phosphate de sodium 50 mM (pH 6,5), ADN de thymus de veau (250 $\mu$g/ml) et formamide déionisé 35%, puis hybridation dans la même solution pendant 48 heures à 42°C avec l'ADN viral marqué par $^{32}$P (2 x 10$^4$ cpm/cm$^2$) et lavage des filtres sous agitation pendant 30 minutes à 50°C dans une solution de Tris-chlorhydrate 60 mM (pH8), EDTA 2 mM, NaCl 300 mM et dodécyl-sulfate de sodium 0,5%, puis pendant 30 minutes à température ambiante dans une solution Tris 3 mM
ou fragments d'ADN correspondant à l'une des régions E1 ; E6 ; E7 ; L1 ; L2 ou à une région intergénique de cet ADN-HPV ou fragment d'ADN correspondant hybridant avec le précédant dans les susdites conditions strictes.

**13.** ADN cloné ou recombinant de HPV 29 (C.N.C.M. n° I-395) ou ADN cloné correspondant de cet ADN cloné qui hybride avec le précédent dans des conditions strictes : chauffage de ces ADNs recombinants, après dénaturation et transfert préalable sur des filtres de transfert pour hybridation, à 80°C pendant 4 heures, préhybridation pendant la nuit à 42°C des filtres dans une solution de Denhardt, 2 x SSC, phosphate de sodium 50 mM (pH, 6,5), ADN de thymus de veau (250 $\mu$g/ml) et formamide déionisé 35%, puis hybridation dans la même solution pendant 48 heures à 42°C avec l'ADN viral marqué par $^{32}$P (2 x 10$^4$ cpm/cm$^2$) et lavage des filtres sous agitation pendant 30 minutes à 50°C dans une solution de Tris-chlorhydrate 60 mM (pH8), EDTA 2 mM, NaCl 300 mM et dodécyl-sulfate de sodium 0,5%, puis pendant 30 minutes à température ambiante dans une solution Tris 3 mM
ou fragments d'ADN correspondant à l'une des régions E1 ; E6 ; E7 ; L1 ; L2 ou à une région intergénique de cet ADN-HPV ou fragment d'ADN correspondant hybridant avec le précédant dans les susdites conditions strictes.

**14.** ADN cloné ou recombinant de HPV 31 (C.N.C.M. n° I-396) ou ADN cloné correspondant de cet ADN cloné qui hybride avec le précédent dans des conditions strictes : chauffage de ces ADNs recombinants, après dénaturation et transfert préalable sur des filtres de transfert pour hybridation, à 80°C pendant 4 heures, préhybridation pendant la nuit à 42°C des filtres dans une solution de Denhardt, 2 x SSC, phosphate de sodium 50 mM (pH 6,5), ADN de thymus de veau (250 $\mu$g/ml) et formamide déionisé 35%, puis hybridation dans la même solution pendant 48 heures à 42'C avec l'ADN viral marqué par $^{32}$P (2 x 10$^4$ cpm/cm$^2$) et lavage des filtres sous agitation pendant 30 minutes à 50°C dans une solution de Tris-chlorhydrate 60 mM (pH8), EDTA 2 mM, NaCl 300 mM et dodécyl-sulfate de sodium 0,5%, puis pendant 30 minutes à température ambiante dans une solution Tris 3 mM
ou fragments d'ADN correspondant à l'une des régions E1 ; E6 ; E7 ; L1 ; L2 ou à une région intergénique de cet ADN-HPV ou fragment d'ADN correspondant hybridant avec le précédant dans les susdites conditions strictes.

15. ADN cloné ou recombinant de HPV 32 (C.N.C.M. n° I-397) ou ADN cloné correspondant de cet ADN cloné qui hybride avec le précédent dans des conditions strictes : chauffage de ces ADNs recombinants, après dénaturation et transfert préalable sur des filtres de transfert pour hybridation, à 80°C pendant 4 heures, préhybridation pendant la nuit à 42°C des filtres dans une solution de Denhardt, 2 x SSC, phosphate de sodium 50 mM (pH, 6,5), ADN de thymus de veau (250 $\mu$g/ml) et formamide déionisé 35%, puis hybridation dans la même solution pendant 48 heures à 42°C avec l'ADN viral marqué par $^{32}$P (2 x 10$^4$ cpm/cm$^2$) et lavage des filtres sous agitation pendant 30 minutes à 50°C dans une solution de Tris-chlorhydrate 60 mM (pH8), EDTA 2 mM, NaCl 300 mM et dodécyl-sulfate de sodium 0,5%, puis pendant 30 minutes à température ambiante dans une solution Tris 3 mM
ou fragments d'ADN correspondant à l'une des régions E1 ; E6 ; E7 ; L1 ; L2 ou à une région intergénique de cet ADN-HPV ou fragment d'ADN correspondant hybridant avec le précédant dans les susdites conditions strictes.

16. ADN cloné ou recombinant de HPV IP2 (C.N.C.M. n° I-450) ou ADN cloné correspondant de cet ADN cloné qui hybride avec le précédent dans des conditions strictes : chauffage de ces ADNs recombinants, après dénaturation et transfert préalable sur des filtres de transfert pour hybridation, à 80°C pendant 4 heures, préhybridation pendant la nuit à 42°C des filtres dans une solution de Denhardt, 2 x SSC, phosphate de sodium 50 mM (pH 6,5), ADN de thymus de veau (250 $\mu$g/ml) et formamide déionisé 35%, puis hybridation dans la même solution pendant 48 heures à 42°C avec l'ADN viral marqué par $^{32}$P (2 x 10$^4$ cpm/cm$^2$) et lavage des filtres sous agitation pendant 30 minutes à 50°C dans une solution de Tris-chlorhydrate 60 mM (pH8), EDTA 2 mM, NaCl 300 mM et dodécyl-sulfate de sodium 0,5%, puis pendant 30 minutes à température ambiante dans une solution Tris 3 mM
ou fragments d'ADN correspondant à l'une des régions E1 ; E6 ; E7 ; L1 ; L2 ou à une région intergénique de cet ADN-HPV ou fragment d'ADN correspondant hybridant avec le précédant dans les susdites conditions strictes.

17. ADN cloné ou recombinant de HPV I4a (C.N.C.M. n° I-382) ou ADN cloné correspondant de cet ADN cloné qui hybride avec le précédent dans des conditions strictes : chauffage de ces ADNs recombinants, après dénaturation et transfert préalable sur des filtres de transfert pour hybridation, à 80°C pendant 4 heures, préhybridation pendant la nuit à 42°C des filtres dans une solution de Denhardt, 2 x SSC, phosphate de sodium 50 mM (pH, 6,5), ADN de thymus de veau (250 $\mu$g/ml) et formamide déionisé 35%, puis hybridation dans la même solution pendant 48 heures à 42°C avec l'ADN viral marqué par $^{32}$P (2 x 10$^4$ cpm/cm$^2$) et lavage des filtres sous agitation pendant 30 minutes à 50°C dans une solution de Tris-chlorhydrate 60 mM (pH8), EDTA 2 mM, NaCl 300 mM et dodécyl-sulfate de sodium 0,5%, puis pendant 30 minutes à température ambiante dans une solution Tris 3 mM
ou fragments d'ADN correspondant à l'une des régions E1 ; E6 ; E7 ; L1 ; L2 ou à une région intergénique de cet ADN-HPV ou fragment d'ADN correspondant hybridant avec le précédant dans les susdites conditions strictes.

18. ADN cloné ou recombinant de HPV I4b (C.N.C.M. n° I-383) ou ADN cloné correspondant de cet ADN cloné qui hybride avec le précédent dans des conditions strictes : chauffage de ces ADNs recombinants, après dénaturation et transfert préalable sur des filtres de transfert pour hybridation, à 80°C pendant 4 heures, préhybridation pendant la nuit à 42°C des filtres dans une solution de Denhardt, 2 x SSC, phosphate de sodium 50 mM (pH, 6,5), ADN de thymus de veau (250 $\mu$g/ml) et formamide déionisé 35%, puis hybridation dans la même solution pendant 48 heures à 42°C avec l'ADN viral marqué par $^{32}$P (2 x 10$^4$ cpm/cm$^2$) et lavage des filtres sous agitation pendant 30 minutes à 50°C dans une solution de Tris-chlorhydrate 60 mM (pH8), EDTA 2 mM, NaCl 300 mM et dodécyl-sulfate de sodium 0,5%, puis pendant 30 minutes à température ambiante dans une solution Tris 3 mM
ou fragments d'ADN correspondant à l'une des régions E1 ; E6 ; E7 ; L1 ; L2 ou à une région intergénique de cet ADN-HPV ou fragment d'ADN correspondant hybridant avec le précédant dans les susdites conditions strictes.

19. Procédé de détection et d'identification de papillomavirus d'un type ou sous-type correspondant à un ADN de l'une quelconque des revendications 10 à 17, éventuellement contenus dans un échantillon biologique, comprenant la réalisation d'un essai d'hybridation avec l'ADN ou fragment d'ADN-HPV correspondant utilisé à titre de sonde, et la détection de l'hybride éventuellement formé entre les ADN-HPVs de l'échantillon biologique et la sonde utilisée.

**Claims**

1. Composition useful for the detection of papillomavirus in a biological medium which contains it, characterized in that it comprises one or several distinct cloned DNA-HPVs selected from the cloned DNAs deposited on November 30, 1984 at the C.N.C.M. (National Collection of Cultures of Micro-organisms of the PASTEUR INSTITUTE of Paris), under the numbers hereafter:

```
HPV 2d  .................. n° I-379
HPV 10bA ................ n° I-380
HPV 10bB ............... n° I-381
HPV 28  ................. n° I-394
HPV 29  ................. n° I-395
HPV 31  ................. n° I-396
HPV 32  ................. n° I-397
IP-2  ................... n° I-450
IP-4  ................... n° I-449
```

- or one or several corresponding cloned DNAs which hybridize with any of those which have been filed at the C.N.C.M., under stringent conditions: heating of these recombinant DNAs, after denaturation and prior transfer on transfer filters for hybridization at 80°C for 4 hours, prehybridization overnight at 42°C of the filters in Denhardt solution, 2 x SSC, 50mM sodium phosphate (pH 6.5), calf thymus DNA (250 $\mu$g/ml) and 35 % deionized formamide, then hybridization in the same solution during 48 hours at 42°C with the $^{32}$P - labeled viral DNA (2 x $10^4$ cpm/cm$^2$) and washing of the filters by shaking over 30 minutes at 50°C in a 60 mM Tris-hydrochloride solution (pH8), 2 mM EDTA, 300 mM NaCl and 0.5% sodium dodecyl sulfate during 30 minutes at ambient temperature in a 3 mM Tris solution.
- or one or several cloned fragments selected among the fragments obtained from the cloned HPV-DNAs filed at the CNCM under the abobe indicated numbers and which correspond to E1-; E6-; E7-; L1-; L2- regions or to intergenic regions of said cloned DNAs;
- or one or several fragments of corresponding cloned DNAs which hybridize with any of the preceding ones under the said stringent conditions.

2. Set or kit comprising a plurality of probes or mixture of distinct probes characterized by nine groups of probes, whereby each of them comprises:
(1) at least the cloned or recombinant DNA of HPV 2d (C.N.C.M. n° I-379);
(2) at least one DNA selected from the cloned or recombinant DNAs of HPV 10b (C.N.C.M. N° I-380 and n° I-381), HPV 28 (C.N.C.M. n° I-394) and HPV 29 (C.N.C.M. n° I-395);
(3) at least one DNA selected from the cloned or recombinant DNAs of HPV 17 (C.N.C.M. n° I-385 and n° I-386), and HPV 24 (C.N.C.M. n° I-392 and n° I-393);
(4) at least one DNA selected from the cloned or recombinant DNAs of HPV 14 (C.N.C.M. N° I-382 and n° I-383), HPV 15 (C.N.C.M. n° I-384), HPV 17 (C.N.C.M. n° I-385 and n° I-386), HPV 19 (C.N.C.M. n° I-387), HPV 20 (C.N.C.M. n° I-388), HPV 21 (C.N.C.M. n° I-389), HPV 22 (C.N.C.M. n° I-390) and HPV 23 (C.N.C.M. n° I-391)
(5) at least one DNA selected from the cloned or recombinant DNAs of HPV 15 (C.N.C.M. n° I-384) and HPV 17 (C.N.C.M. N° I-385 et n° I-386);
(6) the cloned or recombinant DNA of HPV 24 (C.N.C.M. n° I-392 and n° I-393)
(7) at least one DNA selected from the cloned or recombinant DNAs of HPV 14 (C.N.C.M. N° I-382 and n° I-383), and HPV 32 (C.N.C.M. n° I-397) ;

(8) the cloned or recombinant DNA of HPV 31 (C.N.C.M. n° I-396)

(9) the cloned or recombinant DNA of HPV 32 (C.N.C.M. n° I-397);

it been understood

- that each of these recombinant or cloned DNAs can be replaced by a corresponding cloned DNA or DNA fragment which hybridizes with the preceding one under stringent conditions: heating of these recombinant DNAs, after denaturation and prior transfer on transfer filters for hybridization at 80°C for 4 hours, prehybridization overnight at 42°C of the filters in Denhardt solution, 2 x SSC, 50mM sodium phosphate (pH 6.5), calf thymus DNA (250 $\mu$g/ml) and 35 % deionized formamide, then hybridization in the same solution during 48 hours at 42°C with the $^{32}$P - labeled viral DNA (2 x $10^4$ cpm/cm$^2$) and washing of the filters by shaking over 30 minutes at 50°C in a 60 mM Tris-hydrochloride solution (pH8), 2 mM EDTA, 300 mM NaCl and 0.5 % sodium dodecyl sulfate during 30 minutes at ambient temperature in a 3 mM Tris solution,
- that the DNAs of DNA fragments of the nine groups are selected such as to be different one from another under all circumstances, insofar as each of the nine group would be restricted to only one of the DNAs which form that group.

3. Set or kit according to claim 2, characterized in that the groups respectively comprise:

(1) a mixture of cloned or recombinant DNAs of HPV 1, 2d, 4 or a mixture of fragments of said DNAs;

(2) a mixture of cloned or recombinant DNAs of DNA-HPV 3, 10a, 10b, 28 and 29 or a mixture of fragments of said DNAs;

(3) a mixture of cloned or recombinant DNAs of DNA-HPV 5, 17a and 24 or a mixture of fragments of said DNAs;

(4) a mixture of cloned or recombinant DNAs of DNA-HPV 5, 8, 12, 14a, 14b, 19, 20, 21, 22 and 23 or a mixture of fragments of said DNAs;

(5) a mixture of cloned or recombinant DNAs of DNA-HPV 9, 15, 17a and 17b or a mixture of fragments of said DNAs;

(6) a mixture of cloned or recombinant DNAs of DNA-HPV 24 or a mixture of fragments of said DNAs;

(7) a mixture of cloned or recombinant DNAs of DNA-HPV 5, 8, 14b and 32 or a mixture of fragments of said DNAs;

(8) a mixture of cloned or recombinant DNAs of DNA-HPV 18 and 31 or a mixture of fragments of said DNAs;

(9) a cloned or recombinant DNA of HPV-HPV 32.

4. Set or kit according to claim 2 or claim 3, characterized in that group 7) comprises furthermore the cloned or recombinant DNA of HPV IP4 (C.N.C.M. N° I-449) or a cloned or recombinant fragment of said DNA and in that said set or kit comprises a tenth group comprising a mixture of cloned of recombinant DNAs of HPV 16, HPV 18 and IP 2 (C.N.C.M. n° I-450), or a mixture of fragments of said DNAs.

5. Set or kit according to claim 4, characterized in that the fragments brought into play in said groups or mixtures corresponding to the E1; E6; E7; L1; L2 regions, or to intergenic regions of said DNA-HPV fragments.

6. Set or kit according to anyone of claims 2 or 3, applicable to in vitro diagnostics respectively:

(1) of skin or mucous membrane warts (especially verruca and plantar warts); differential diagnosis of epidermodysplasia verruciformis;

(2) of plane warts or intermediary skin or mucous membrane warts; intra-epithelial neoplasms and skin cancers; differential diagnosis of epidermodysplasia verruciformis;

(3) of epidermodysplasia verruciformis; intra-epithelial neoplasms and skin cancers;

(4) of epidermodysplasia verruciformis;

(5) of epidermodysplasia verruciformis;

(6) of epidermodysplasia verruciformis;

(7) of skin cancers from epidermodysplasia verruciformis; intra-epithelial neoplasias and skin cancers;

(8) of oral epithelial hyperplasia; differential diagnosis of oral epithelial neoplasms;

(9) of intra-epithelial neoplasms and skin cancers.

**7.** Set or kit according to claim 4 or or to claim 5, applicable to respective in vitro diagnosis:

(1) of skin or mucous membrane warts (especially verruca and plantar warts); differential diagnosis of epidermodysplasia verruciformis;

(2) of plane warts or intermediary skin or mucous membrane warts; intra-epithelial neoplasms and skin cancers; differential diagnosis of epidermodysplasia verruciformis;

(3) of epidermodysplasia verruciformis; intra-epithelial neoplasms and skin cancers;

(4) of epidermodysplasia verruciformis;

(5) of epidermodysplasia verruciformis;

(6) of epidermodysplasia verruciformis;

(7) of skin cancers from epidermodysplasia verruciformis; intra-epithelial neoplasias and skin cancers;

(8) of oral epithelial hyperplasia; differential diagnosis of oral epithelial neoplasms;

(9) of intra-epithelial neoplasms and skin cancers.

(10) of genital neoplasms, and particularly, of uterus-cancers.

**8.** Process of in vitro detection and identification of papillomavirus contained in a biological sample comprising the carrying out of distinct hybridization tests with the cloned or recombinant DNA-HPVs or the fragments of the DNA-HPVs of these sets or kits, according to anyone of claims 2 to 7 and the detection of those DNA-HPVs or cloned fragments which give rise to a preferential hybridization with the DNA-HPVs contained in the the biological sample.

**9.** Process according to claim 8, characterized in that the hybridization tests are carried out with the whole cloned DNAs or the DNA fragments corresponding to the E6 or E7 regions under stringent hybridization conditions.

**10.** Cloned or recombinant DNA of HPV 2d (C.N.C.M. n° I-379) or corresponding cloned DNA of said cloned DNA which hybridizes with the preceding one under stringent conditions: heating of said recombinant DNAs after denaturation and prior transfer on transfer filter for hybridization at 80°C for 4 hours, prehybridization overnight at 42°C of the filters in Denhardt solution, 2 x SSC, 50mM sodium phosphate (pH 6.5), calf thymus DNA (250 $\mu$g/ml) and 35 % deionized formamide, then hybridization in the same solution during 48 hours at 42°C with the $^{32}$P - labeled viral DNA (2 x 10$^4$ cpm/cm$^2$) and washing of the filters by shaking over 30 minutes at 50°C in a 60 mM Tris-hydrochloride solution (pH8), 2 mM EDTA, 300 mM NaCl and 0.5% sodium dodecyl sulfate during 30 minutes at ambient temperature in a 3 mM Tris solution.

or DNA fragments corresponding to the E1; E6; E7; L1; L2 regions or to one intergenic region of said DNA-HPV or corresponding DNA fragment which hybridizes with the preceding one under said stringent conditions.

**11.** Cloned or recombinant DNA of HPV 10b (C.N.C.M. n° I-380 and n° I-381) or corresponding cloned DNA of said cloned DNA which hybridizes with the preceding one under stringent conditions: heating of said recombinant DNAs after denaturation and prior transfer on transfer filters for hybridization at 80°C for 4 hours, prehybridization overnight at 42°C of the filters in Denhardt solution, 2 x SSC, 50mM sodium phosphate (pH 6.5), calf thymus DNA (250 $\mu$g/ml) and 35 % deionized formamide, then hybridization in the same solution during 48 hours at 42°C with the $^{32}$P - labeled viral DNA (2 x 10$^4$ cpm/cm$^2$) and washing of the filters by shaking over 30 minutes at 50°C in a 60 mM Tris-hydrochloride solution (pH8), 2 mM EDTA, 300 mM NaCl and 0.5% sodium dodecyl sulfate during 30 minutes at ambient temperature in a 3 mM Tris solution.

or DNA fragments corresponding to the E1; E6; E7; L1; L2 regions or to one intergenic region of said DNA-HPV or corresponding DNA fragment which hybridizes with the preceding one under said stringent conditions.

**12.** Cloned or recombinant DNA of HPV 28 (C.N.C.M. n° I-394) or corresponding cloned DNA of said cloned DNA which hybridizes with the preceding one under stringent conditions: heating of said recombinant DNAs after denaturation and prior transfer on transfer filters for hybridization at 80°C for 4 hours, prehybridization overnight at 42°C of the filters in Denhardt solution, 2 x SSC, 50mM sodium phosphate (pH 6.5), calf thymus DNA (250 $\mu$g/ml) and 35 % deionized formamide, then hybridization in the same solution during 48 hours at 42°C with the $^{32}$P - labeled viral DNA (2 x 10$^4$ cpm/cm$^2$) and washing of the filters by shaking over 30 minutes at 50°C in a 60 mM Tris-hydrochloride solution (pH8), 2 mM EDTA, 300 mM NaCl and 0.5% sodium dodecyl sulfate during 30 minutes at ambient temperature in a 3 mM Tris solution.

or DNA fragments corresponding to the E1; E6; E7; L1; L2 regions or to one intergenic region of said DNA-HPV or corresponding DNA fragment which hybridizes with the preceding one under said stringent conditions.

13. Cloned or recombinant DNA of HPV 29 (C.N.C.M. n° I-395) or corresponding cloned DNA of said cloned DNA which hybridizes with the preceding one under stringent conditions: heating of said recombinant DNAs after denaturation and prior transfer on transfer filters for hybridization at 80°C for 4 hours, prehybridization overnight at 42°C of the filters in Denhardt solution, 2 x SSC, 50mM sodium phosphate (pH 6.5), calf thymus DNA (250 $\mu$g/ml) and 35 % deionized formamide, then hybridization in the same solution during 48 hours at 42°C with the $^{32}$P - labeled viral DNA (2 x 10$^4$ cpm/cm$^2$) and washing of the filters by shaking over 30 minutes at 50°C in a 60 mM Tris-hydrochloride solution (pH8), 2 mM EDTA, 300 mM NaCl and 0.5% sodium dodecyl sulfate during 30 minutes at ambient temperature in a 3 mM Tris solution.

or DNA fragments corresponding to the E1; E6; E7; L1; L2 regions or to one intergenic region of said DNA-HPV or corresponding DNA fragment which hybridizes with the preceding one under said stringent conditions.

14. Cloned or recombinant DNA of HPV 31 (C.N.C.M. n° I-396) or corresponding cloned DNA of said cloned DNA which hybridizes with the preceding one under stringent conditions: heating of said recombinant DNAs after denaturation and prior transfer on transfer filters for hybridization at 80°C for 4 hours, prehybridization overnight at 42°C of the filters in Denhardt solution, 2 x SSC, 50mM sodium phosphate (pH 6.5), calf thymus DNA (250 $\mu$g/ml) and 35 % deionized formamide, then hybridization in the same solution during 48 hours at 42°C with the $^{32}$P - labeled viral DNA (2 x 10$^4$ cpm/cm$^2$) and washing of the filters by shaking over 30 minutes at 50°C in a 60 mM Tris-hydrochloride solution (pH8), 2 mM EDTA, 300 mM NaCl and 0.5% sodium dodecyl sulfate during 30 minutes at ambient temperature in a 3 mM Tris solution.

or DNA fragments corresponding to the E1; E6; E7; L1; L2 regions or to one intergenic region of said DNA-HPV or corresponding DNA fragment which hybridizes with the preceding one under said stringent conditions.

15. Cloned or recombinant DNA of HPV 32 (C.N.C.M. n° I-397) or corresponding cloned DNA of said cloned DNA which hybridizes with the preceding one under stringent conditions: heating of said recombinant DNAs after denaturation and prior transfer on transfer filters for hybridization at 80°C for 4 hours, prehybridization overnight at 42°C of the filters in Denhardt solution, 2 x SSC, 50mM sodium phosphate (pH 6.5), calf thymus DNA (250 $\mu$g/ml) and 35 % deionized formamide, then hybridization in the same solution during 48 hours at 42°C with the $^{32}$P - labeled viral DNA (2 x 10$^4$ cpm/cm$^2$) and washing of the filters by shaking over 30 minutes at 50°C in a 60 mM Tris-hydrochloride solution (pH8), 2 mM EDTA, 300 mM NaCl and 0.5% sodium dodecyl sulfate during 30 minutes at ambient temperature in a 3 mM Tris solution.

or DNA fragments corresponding to the E1; E6; E7; L1; L2 regions or to one intergenic region of said DNA-HPV or corresponding DNA fragment which hybridizes with the preceding one under said stringent conditions.

16. Cloned or recombinant DNA of HPV IP2 (C.N.C.M. n° I-450) or corresponding cloned DNA of said cloned DNA which hybridizes with the preceding one under stringent conditions: heating of said recombinant DNAs after denaturation and prior transfer on transfer filters for hybridization at 80°C for 4 hours, prehybridization overnight at 42°C of the filters in Denhardt solution, 2 x SSC, 50mM sodium phosphate (pH 6.5), calf thymus DNA (250 $\mu$g/ml) and 35 % deionized formamide, then hybridization in the same solution during 48 hours at 42°C with the $^{32}$P - labeled viral DNA (2 x 10$^4$ cpm/cm$^2$) and washing of the filters by shaking over 30 minutes at 50°C in a 60 mM Tris-hydrochloride solution (pH8), 2 mM EDTA, 300 mM NaCl and 0.5% sodium dodecyl sulfate during 30 minutes at ambient temperature in a 3 mM Tris solution.

or DNA fragments corresponding to the E1; E6; E7; L1; L2 regions or to one intergenic region of said DNA-HPV or corresponding DNA fragment which hybridizes with the preceding one under said stringent conditions.

27

EP 0 192 001 B1

17. Cloned or recombinant DNA of HPV I4a (C.N.C.M. n° I-382) or corresponding cloned DNA of said cloned DNA which hybridizes with the preceding one under stringent conditions: heating of said recombinant DNAs after denaturation and prior transfer on transfer filters for hybridization at 80°C for 4 hours, prehybridization overnight at 42°C of the filters in Denhardt solution, 2 x SSC, 50mM sodium phosphate (pH 6.5), calf thymus DNA (250 $\mu$g/ml) and 35 % deionized formamide, then hybridization in the same solution during 48 hours at 42°C with the $^{32}$P - labeled viral DNA (2 x $10^4$ cpm/cm$^2$) and washing of the filters by shaking over 30 minutes at 50°C in a 60 mM Tris-hydrochloride solution (pH8), 2 mM EDTA, 300 mM NaCl and 0.5% sodium dodecyl sulfate during 30 minutes at ambient temperature in a 3 mM Tris solution.

or DNA fragments corresponding to the E1; E6; E7; L1; L2 regions or to one intergenic region of said DNA-HPV or corresponding DNA fragment which hybridizes with the preceding one under said stringent conditions.

18. Cloned or recombinant DNA of HPV I4b (C.N.C.M. n° I-383) or corresponding cloned DNA of said cloned DNA which hybridizes with the preceding one under stringent conditions: heating of said recombinant DNAs after denaturation and prior transfer on transfer filters for hybridization at 80°C for 4 hours, prehybridization overnight at 42°C of the filters in Denhardt solution, 2 x SSC, 50mM sodium phosphate (pH 6.5), calf thymus DNA (250 $\mu$g/ml) and 35 % deionized formamide, then hybridization in the same solution during 48 hours at 42°C with the $^{32}$P - labeled viral DNA (2 x $10^4$ cpm/cm$^2$) and washing of the filters by shaking over 30 minutes at 50°C in a 60 mM Tris-hydrochloride solution (pH8), 2 mM EDTA, 300 mM NaCl and 0.5% sodium dodecyl sulfate during 30 minutes at ambient temperature in a 3 mM Tris solution.

or DNA fragments corresponding to the E1; E6; E7; L1; L2 regions or to one intergenic region of said DNA-HPV or corresponding DNA fragment which hybridizes with the preceding one under said stringent conditions.

19. Process of detection or identification of papillomavirus of a type or subtype corresponding to a DNA according to anyone of claims 10 to 17, possibly contained in a biological sample, comprising the carrying out of a hybridization test with the corresponding DNA or DNA-HPV fragment used in a form of a probe and the detection of the hybrid possibly formed between the DNA-HPV of the biological sample and the probe which has been used.

**Patentansprüche**

1. Mittel, verwendbar für den Nachweis des Papillomavirus in einem dieses enthaltenden biologischen Milieu, **dadurch gekennzeichnet**, daß es eine oder mehrere unterschiedliche clonierte HPV-DNAs enthält, die ausgewählt sind aus den clonierten DNAs, die am 30. November 1984 bei C.N.C.M. (Collection Nationale de Cultures de Microorganismes de l'Institut Pasteur de Paris) unter den folgenden Nummern hinterlegt wurden:

```
HPV 2d .............. Nr. I-379
HPV 10bA ........... Nr. I-380
HPV 10bB .......... Nr. I-381
HPV 28 ............ Nr. I-394
HPV 29 ........... Nr. I-395
HPV 31 ............ Nr. I-396
HPV 32 ............ Nr. I-397
IP-2 .............. Nr. I-450
IP-4 .............. Nr. I-449
```

- oder ein oder mehrere entsprechende clonierte DNAs, die mit einer dieser bei C.N.C.M. hinterlegten unter den folgenden stringenten Bedingungen hybridisieren: Erwärmung dieser rekombinanten DNAs nach Denaturierung und vorhergehendem Transfer auf Transferfilter für die

28

Hybridisierung bei 80°C während 4 Stunden, Vorhybridisierung der Filter über Nacht bei 42°C in einer Denhardt-Lösung, 2 x SSC, 50 mM Natriumphosphat (pH 6,5), Kalbsthymus-DNA (250 $\mu$g/ml) und 35 % entionisiertes Formamid, danach Hybridisierung in der gleichen Lösung während 48 Stunden bei 42°C mit viraler DNA, die mit $^{32}$P (2 x 10$^4$ cpm/cm$^2$) markiert ist, und Waschen der Filter unter Bewegung während 30 Minuten bei 50°C in einer Lösung von 60 mM Tris-Chlorhydrat (pH 8), 2 mM EDTA, 300 mM NaCl und 0,5% Natriumdodecylsulfat, danach während 30 Minuten bei Raumtemperatur in einer Lösung von 3mM Tris,

- oder eines oder mehrere clonierte Fragmente, ausgewählt aus den Fragmenten, die von den clonierten HPV-DNAs stammen, die bei der C.N.C.M. unter den vorstehend angegebenen Nummern hinterlegt wurden und die den Bereichen E1, E6, E7, L1, L2 oder intergenischen Bereichen dieser clonierten DNAs entsprechen,

- oder eines oder mehrere entsprechende clonierte DNA-Fragmente, die mit einem der vorangehenden unter den vorstehend angegebenen genauen Bedingungen hybridisieren.

2. Mittel oder Kit, enthaltend eine Vielzahl von Sonden oder Gemischen von verschiedenen Sonden, gekennzeichnet durch neun Sondengruppen, von denen jede enthält:

(1) mindestens die clonierte oder rekombinante DNA von HPV 2d (C.N.C.M. Nr. I-379);

(2) mindestens eine DNA, ausgewählt aus den clonierten oder rekombinanten DNAs von HPV 10b (C.N.C.M. Nr. I-380 und Nr. I-381), HPV 28 (C.N.C.M. Nr. I-394) und HPV 29 (C.N.C.M. Nr. I-395);

(3) mindestens eine DNA, ausgewählt aus den clonierten oder rekombinanten DNAs von HPV 17 (C.N.C.M. Nr. I-385 und Nr. I-386) und HPV 24 (C.N.C.M. Nr. I-392 und Nr. I-393);

(4) mindestens eine DNA, ausgewählt aus den clonierten oder rekombinanten DNAs von HPV 14 (C.N.C.M. Nr. I-382 und Nr. I-383), HPV 15 (C.N.C.M. Nr. I-384), HPV 17 (C.N.C.M. Nr. I-385 und Nr. I-386), HPV 19 (C.N.C.M. Nr. I-387), HPV 20 (C.N.C.M. Nr. I-388), HPV 21 (C.N.C.M. Nr. I-389), HPV 22 (C.N.C.M. Nr. I-390) und HPV 23 (C.N.C.M. Nr. I-391);

(5) mindestens eine DNA, ausgewählt aus den clonierten oder rekombinanten DNAs von HPV 15 (C.N.C.M. Nr. I-384) und HPV 17 (C.N.C.M. Nr. I-385 und Nr. I-386);

(6) die clonierte oder rekombinante DNA von HPV 24 (C.N.C.M. Nr. I-392 und Nr. I-393);

(7) mindestens eine DNA, ausgewählt aus den clonierten oder rekombinanten DNAs von HPV 14 (C.N.C.M. Nr. I-382 und Nr. I-383) und HPV 32 (C.N.C.M. Nr. I-397);

(8) die clonierte oder rekombinante DNA von HPV 31 (C.N.C.M. Nr. I-396);

(9) die clonierte oder rekombinante DNA von HPV 32 (C.N.C.M. Nr. I-397);

mit der Maßgabe,

- daß jede dieser rekombinanten oder clonierten DNAs durch eine entsprechende clonierte DNA oder durch ein DNA-Fragment ersetzt werden kann, das mit der vorangehenden unter den folgenden stringenten Bedingungen hybridisiert: Erwärmung dieser rekombinanten DNAs nach Denaturierung und vorangehendem Transfer auf Transferfilter für die Hybridisierung bei 80°C während 4 Stunden, Vorhybridisierung der Filter bei 42° über Nacht in einer Denhardt-Lösung, 2 x SSC, 50 mM Natriumphosphat (pH 6,5), Kalbsthymus-DNA (250 $\mu$g/ml) und 35 % entionisiertes Formamid, danach Hybridisierung in der gleichen Lösung während 48 Stunden bei 42°C mit viraler DNA, die mit $^{32}$P (2 x 10$^4$ cpm/cm$^2$) markiert ist, und Waschen der Filter unter Bewegung während 30 Minuten bei 50°C in einer Lösung von 60 mM Tris-Chlorhydrat (pH 8), 2 mM EDTA, 300 mM NaCl und Natriumdodecylsulfat, anschließend während 30 Minuten bei Raumtemperatur in einer 3 mM Tris-Lösung,

- daß die DNAs oder DNA-Fragmente der neun Gruppen so ausgewählt sind, daß sie unter allen Umständen voneinander verschieden sind, in dem Maß, in dem jede der neun Gruppen auf eine einzige der sie ausmachenden DNAs reduziert ist.

3. Mittel oder Kit gemäß Anspruch 2, dadurch gekennzeichnet, daß sie folgende Gruppen enthalten:

(1) ein Gemisch von clonierten oder rekombinanten DNAs v0n HPV 1, 2d, 4 oder ein Gemisch von Fragmenten dieser DNAs;

(2) ein Gemisch von clonierten oder rekombinanten DNAs von DNA-HPV 3, 10a, 10b, 28 und 29 oder ein Gemisch von Fragmenten dieser DNAs;

(3) ein Gemisch von clonierten oder rekombinanten DNAs von DNA-HPV 5, 17a und 24 oder ein Gemisch von Fragmenten dieser DNAs;

(4) ein Gemisch von clonierten oder rekombinanten DNAs von DNA-HPV 5, 8, 12, 14a, 14b, 19, 20, 21, 22 und 23 oder ein Gemisch von Fragmenten dieser DNAs;

(5) ein Gemisch von clonierten oder rekombinanten DNAs von DNA-HPVs 9, 15, 17a und 17b oder ein Gemisch von Fragmenten dieser DNAs;

(6) eine clonierte oder rekombinante DNA von DNA-HVP24 oder ein Gemisch von Fragmenten dieser DNA;

(7) ein Gemisch von clonierten oder rekombinanten DNAs von DNA-HPVs 5, 8, 14 und 32 oder ein Gemisch von Fragmenten dieser DNAs;

(8) ein Gemisch clonierter oder rekombinanter DNAs von DNA-HPVs 18 und 31 oder ein Gemisch der Fragmente dieser DNAs;

(9) eine clonierte oder rekombinante DNA von DNA-HPV 32.

4. Mittel oder Kit gemäß Anspruch 2 oder Anspruch 3, dadurch gekennzeichnet, daß die Gruppe 7) eine weitere clonierte oder rekombinante DNA von HPV IP-4 (C.N.C.M. Nr. I-449) oder ein cloniertes oder rekombinantes Fragment dieser DNA enthält, und daß dieses Mittel oder Kit eine zehnte Gruppe enthält, die ein Gemisch aus clonierten oder rekombinanten DNAs von HPV 16, HPV 18 und IP-2 (C.N.C.M. Nr. I-450) oder ein Gemisch der Fragmente dieser DNAs enthält.

5. Mittel oder Kit gemäß Anspruch 4, dadurch gekennzeichnet, daß die in den Gruppen oder Gemischen verwendeten Fragmente den Bereichen E1, E6, E7, L1, L2 oder den intergenischen Bereichen dieser DNA-HPV-Fragmente entsprechen.

6. Mittel oder Kit gemäß einem der Ansprüche 2 oder 3, für die jeweilige in vitro-Diagnose von:

(1) Haut- oder Schleimhautwarzen (insbesondere gewöhnliche Warzen oder Warzen der Fußsohle); Differentialdiagnose von warzenförmiger Epidermodysplasie;

(2) flache oder intermediäre Haut- oder Schleimhautwarzen; intraepitheliale Neoplasien und Hautkrebs, Differentialdiagnose von warzenförmiger Epidermodysplasie;

(3) warzenförmige Epidermodysplasie; intraepitheliale Neoplasien und Hautkrebs;

(4) warzenförmige Epidermodysplasie;

(5) warzenförmige Epidermodysplasie;

(6) warzenförmige Epidermodysplasie;

(7) aus warzenförmiger Epidermodysplasie entstehender Hautkrebs; intraepitheliale Neoplasien und Hautkrebs;

(8) fokale Hyperplasie der Mundschleimhaut; Differentialdiagnose von intraepithelialen oralen Neoplasien;

(9) intraepitheliale Neoplasien und Hautkrebs.

7. Mittel oder Kit gemäß Anspruch 4 oder Anspruch 5, für die jeweilige in vitro-Diagnose von:

(1) Haut- oder Schleimhautwarzen (insbesondere gewöhnliche Warzen oder Warzen der Fußsohle); Differentialdiagnose von warzenförmiger Epidermodysplasie;

(2) flache Warzen oder intermediäre Haut- oder Schleimhautwarzen; intraepitheliale Neoplasien und Hautkrebs; Differentialdiagnose von warzenförmiger Epidermodysplasie;

(3) warzenförmige Epidermodysplasie; intraepitheliale Neoplasien und Hautkrebs;

(4) warzenförmige Epidermodysplasie;

(5) warzenförmige Epidermodysplasie;

(6) warzenförmige Epidermodysplasie;

(7) aus warzenförmiger Epidermodysplasie entstehender Hautkrebs; intraepitheliale Neoplasien und Hautkrebs;

(8) fokale Hyperplasie der Mundschleimhaut; Differentialdiagnose von intraepithelialen oralen Neoplasien;

(9) intraepitheliale Neoplasien und Hautkrebs;

(10) Genital-Neoplasien und insbesondere Gebärmutterkrebs.

8. Verfahren zum Nachweis und zur in vitro-Identifizierung des in einer biologischen Flüssigkeit enthaltenen Papillomavirus, umfassend die Durchführung verschiedener Hybridisierungstests mit den clonierten oder rekombinanten DNA-HPVs oder den Fragmenten von DNA-HPVs gemäß den Mitteln oder Kits der Ansprüche 2 bis 7 und Nachweis dieser clonierten DNA-HPVs oder Fragmente, die eine bevorzugte Hybridisierung mit den in der biologischen Flüssigkeit enthaltenen DNA-HPVs ergeben.

9. Verfahren nach Anspruch 8, dadurch gekennzeichnet, daß die Hybridisierungstests unter den genauen Hybridisierungsbedingungen mit den vollständigen clonierten DNAs oder DNA-Fragmenten, die den Bereichen E6 oder E7 entsprechen, gemacht werden.

10. Clonierte oder rekombinante DNA von HPV 2d (C.N.C.M. Nr. I-379) oder clonierte DNA, die der clonierten DNA entspricht, die mit der vorangehenden unter den folgenden stringenten Bedingungen hybridisiert: Erwärmung dieser rekombinanten DNAs nach Denaturierung und vorangehendem Transfer auf die Transferfilter für die Hybridisierung bei 80°C während 4 Stunden, Vorhybridisierung der Filter bei 42°C über Nacht in einer Denhardt-Lösung, 2 x SSC, 50 mM Natriumphosphat (pH 6,5), Kalbsthymus-DNA (250 $\mu$g/ml) und 35 % entionisiertes Formamid, danach Hybridisierung in der gleichen Lösung während 48 Stunden bei 42°C mit viraler DNA, die mit $^{32}$P (2 x 10$^4$ cpm/cm$^2$) markiert ist, und Waschen der Filter unter Bewegung während 30 Minuten bei 50°C in einer Lösung von 60 mM Tris-Chlorhydrat (pH 8), 2 mM EDTA, 300 mM NaCl und 0,5% Natriumdodecylsulfat, danach während 30 Minuten bei Raumtemperatur in einer Lösung von 3 mM Tris,
oder DNA-Fragmente, die einem der Bereiche E1, E6, E7, L1, L2 oder einem intergenischen Bereich dieser DNA-HPV entsprechen, oder ein DNA-Fragment, das einer DNA entspricht, die mit dem vorangehenden unter den angegebenen stringenten Bedingungen hybridisiert.

11. Clonierte oder rekombinante DNA von HPV 10bd (C.N.C.M. Nr. I-380 und Nr. I-381) oder clonierte DNA, die der clonierten DNA entspricht, die mit der vorangehenden unter den folgenden stringenten Bedingungen hybridisiert: Erwärmung dieser rekombinanten DNAs nach Denaturierung und vorangehendem Transfer auf die Transferfilter für die Hybridisierung bei 80°C während 4 Stunden, Vorhybridisierung der Filter bei 42°C über Nacht in einer Denhardt-Lösung, 2 x SSC, 50 mM Natriumphosphat (pH 6,5), Kalbsthymus-DNA (250 $\mu$g/ml) und 35 % entionisiertes Formamid, danach Hybridisierung in der gleichen Lösung während 48 Stunden bei 42°C mit viraler DNA, die mit $^{32}$P (2 x 10$^4$ cpm/cm$^2$) markiert ist, und Waschen der Filter unter Bewegung während 30 Minuten bei 50°C in einer Lösung von 60 mM Tris-Chlorhydrat (pH 8), 2 mM EDTA, 300 mM NaCl und 0,5% Natriumdodecylsulfat, danach während 30 Minuten bei Raumtemperatur in einer Lösung von 3 mM Tris,
oder DNA-Fragmente, die einem der Bereiche E1, E6, E7, L1, L2 oder einem intergenischen Bereich dieser DNA-HPV entsprechen, oder ein DNA-Fragment, das einer DNA entspricht, die mit dem vorangehenden unter den angegebenen stringenten Bedingungen hybridisiert.

12. Clonierte oder rekombinante DNA von HPV 28 (C.N.C.M. Nr. I-394) oder clonierte DNA, die der clonierten DNA entspricht, die mit der vorangehenden unter den folgenden stringenten Bedingungen hybridisiert: Erwärmung dieser rekombinanten DNAs nach Denaturierung und vorangehendem Transfer auf die Transferfilter für die Hybridisierung bei 80°C während 4 Stunden, Vorhybridisierung der Filter bei 42°C über Nacht in einer Denhardt-Lösung, 2 x SSC, 50 mM Natriumphosphat (pH 6,5), Kalbsthymus-DNA (250 $\mu$g/ml) und 35 % entionisiertes Formamid, danach Hybridisierung in der gleichen Lösung während 48 Stunden bei 42°C mit viraler DNA, die mit $^{32}$P (2 x 10$^4$ cpm/cm$^2$) markiert ist, und Waschen der Filter unter Bewegung während 30 Minuten bei 50°C in einerLösung von 60 mM Tris-Chlorhydrat (pH 8), 2 mM EDTA, 300 mM NaCl und 0,5% Natriumdodecylsulfat, danach während 30 Minuten bei Raumtemperatur in einer Lösung von 3 mM Tris,
oder DNA-Fragmente, die einen der Bereiche E1, E6, E7, L1, L2 oder einem intergenischen Bereich dieser DNA-HPV entsprechen, oder ein DNA-Fragment, das einer DNA entspricht, die mit dem vorangehenden unter den angegebenen stringenten Bedingungen hybridisiert.

13. Clonierte oder rekombinante DNA von HPV 29 (C.N.C.M. Nr. I-395) oder clonierte DNA, die der clonierten DNA entspricht, die mit der vorangehenden unter den folgenden stringenten Bedingungen hybridisiert: Erwärmung dieser rekombinanten DNAs nach Denaturierung und vorangehendem Transfer auf die Transferfilter für die Hybridisierung bei 80°C während 4 Stunden, Vorhybridisierung der Filter bei 42°C über Nacht in einer Denhardt-Lösung, 2 x SSC, 50 mM Natriumphosphat (pH 6,5), Kalbsthymus-DNA (250 $\mu$g/ml) und 35 % entionisiertes Formamid, danach Hybridisierung in der gleichen Lösung während 48 Stunden bei 42°C mit viraler DNA, die mit $^{32}$P (2 x 10$^4$ cpm/cm$^2$) markiert ist, und Waschen der Filter unter Bewegung während 30 Minuten bei 50°C in einer Lösung von 60 mM Tris-Chlorhydrat (pH 8), 2 mM EDTA, 300 mM NaCl und 0,5% Natriumdodecylsulfat, danach während 30 Minuten bei Raumtemperatur in einer Lösung von 3 mM Tris,
oder DNA-Fragmente, die einem der Bereiche E1, E6, E7, L1, L2 oder einem intergenischen Bereich dieser DNA-HPV entsprechen, oder ein DNA-Fragment, das einer DNA entspricht, die mit dem vorangehenden unter den angegebenen stringenten Bedingungen hybridisiert.

**14.** Clonierte oder rekombinante DNA von HPV 31 (C.N.C.M. Nr. I-396) oder clonierte DNA, die der clonierten DNA entspricht, die mit der vorangehenden unter den folgenden stringenten Bedingungen hybridisiert: Erwärmung dieser rekombinanten DNAs nach Denaturierung und vorangehendem Transfer auf die Transferfilter für die Hybridisierung bei 80°C während 4 Stunden, Vorhybridisierung der Filter bei 42°C über Nacht in einer Denhardt-Lösung, 2 x SSC, 50 mM Natriumphosphat (pH 6,5), Kalbsthymus-DNA (250 $\mu$g/ml) und 35 % entionisiertes Formamid, danach Hybridisierung in der gleichen Lösung während 48 Stunden bei 42°C mit viraler DNA, die mit $^{32}$P (2 x 10$^4$ cpm/cm$^2$) markiert ist, und Waschen der Filter unter Bewegung während 30 Minuten bei 50°C in einer Lösung von 60 mM Tris-Chlorhydrat (pH 8), 2 mM EDTA, 300 mM NaCl und 0,5% Natriumdodecylsulfat, danach während 30 Minuten bei Raumtemperatur in einer Lösung Von 3 mM Tris,
oder DNA-Fragmente, die einem der Bereiche E1, E6, E7, L1, L2 oder einem intergenischen Bereich dieser DNA-HPV entsprechen, oder ein DNA-Fragment, das einer DNA entspricht, die mit dem vorangehenden unter den angegebenen stringenten Bedingungen hybridisiert.

**15.** Clonierte oder rekombinante DNA von HPV 32 (C.N.C.M. Nr. I-397) oder clonierte DNA, die der clonierten DNA entspricht, die mit der vorangehenden unter den folgenden Bedingungen hybridisiert: Erwärmung dieser rekombinanten DNAs nach Denaturierung und vorangehendem Transfer auf die Transferfilter für die Hybridisierung bei 80°C während 4 Stunden, Vorhybridisierung der Filter bei 42°C über Nacht in einer Denhardt-Lösung, 2 x SSC, 50 mM Natriumphosphat (pH 6,5), Kalbsthymus-DNA (250 $\mu$g/ml) und 35 % entionisiertes Formamid, danach Hybridisierung in der gleichen Lösung während 48 Stunden bei 42°C mit viraler DNA, die mit $^{32}$P (2 x 10$^4$ cpm/cm$^2$) markiert ist, und Waschen der Filter unter Bewegung während 30 Minuten bei 50°C in einer Lösung von 60 mM Tris-Chlorhydrat (pH 8), 2 mM EDTA, 300 mM NaCl und 0,5% Natriumdodecylsulfat, danach während 30 Minuten bei Raumtemperatur in einer Lösung von 3 mM Tris,
oder DNA-Fragmente, die einem der Bereiche E1, E6, E7, L1, L2 oder einem intergenischen Bereich dieser DNA-HPV entsprechen, oder ein DNA-Fragment, das einer DNA entspricht, die mit dem vorangehenden unter den angegebenen stringenten Bedingungen hybridisiert.

**16.** Clonierte oder rekombinante DNA von HPV IP2 (C.N.C.M. Nr. I-450) oder clonierte DNA, die der clonierten DNA entspricht, die mit der vorangehenden unter den folgenden stringenten Bedingungen hybridisiert: Erwärmung dieser rekombinanten DNAs nach Denaturierung und vorangehendem Transfer auf die Transferfilter für die Hybridisierung bei 80°C während 4 Stunden, Vorhybridisierung der Filter bei 42°C über Nacht in einer Denhardt-Lösung, 2 x SSC, 50 mM Natriumphosphat (pH 6,5), Kalbsthymus-DNA (250 $\mu$g/ml) und 35 % entionisiertes Formamid, danach Hybridisierung in der gleichen Lösung während 48 Stunden bei 42°C mit viraler DNA, die mit $^{32}$P (2 x 10$^4$ cpm/cm$^2$) markiert ist, und Waschen der Filter unter Bewegung während 30 Minuten bei 50°C in einer Lösung von 60 mM Tris-Chlorhydrat (pH 8), 2 mM EDTA, 300 mM NaCl und 0,5% Natriumdodecylsulfat, danach während 30 Minuten bei Raumtemperatur in einer Lösung von 3 mM Tris,
oder DNA-Fragmente, die einen der Bereiche E1, E6, E7, L1, L2 oder einem intergenischen Bereich dieser DNA-HPV entsprechen, oder ein DNA-Fragment, das einer DNA entspricht, die mit dem vorangehenden unter den angegebenen stringenten Bedingungen hybridisiert.

**17.** Clonierte oder rekombinante DNA von HPV I4a (C.N.C.M. Nr. I-382) oder clonierte DNA, die der clonierten DNA entspricht, die mit der vorangehenden unter den folgenden stringenten Bedingungen hybridisiert: Erwärmung dieser rekombinanten DNAs nach Denaturierung und vorangehendem Transfer auf die Transferfilter für die Hybridisierung bei 80°C während 4 Stunden, Vorhybridisierung der Filter bei 42°C über Nacht in einer Denhardt-Lösung, 2 x SSC, 50 mM Natriumphosphat (pH 6,5), Kalbsthymus-DNA (250 $\mu$g/ml) und 35 % entionisiertes Formamid, danach Hybridisierung in der gleichen Lösung während 48 Stunden bei 42°C mit viraler DNA, die mit $^{32}$P (2 x 10$^4$ cpm/cm$^2$) markiert ist, und Waschen der Filter unter Bewegung während 30 Minuten bei 50°C in einer Lösung von 60 mM Tris-Chlorhydrat (pH 8), 2 mM EDTA, 300 mM NaCl und 0,5% Natriumdodecylsulfat, danach während 30 Minuten bei Raumtemperatur in einer Lösung von 3 mM Tris,
oder DNA-Fragmente, die einem der Bereiche E1, E6, E7, L1, L2 oder einem intergenischen Bereich dieser DNA-HPV entsprechen, oder ein DNA-Fragment, das einer DNA entspricht, die mit dem vorangehenden unter den angegebenen stringenten Bedingungen hybridisiert.

**18.** Clonierte oder rekombinante DNA von HPV I4b (C.N.C.M. Nr. I-383) oder clonierte DNA, die der clonierten DNA entspricht, die mit der vorangehenden unter den folgenden stringenten Bedingungen

32

hybridisiert: Erwärmung dieser rekombinanten DNAs nach Denaturierung und vorangehendem Transfer auf die Transferfilter für die Hybridisierung bei 80°C während 4 Stunden, Vorhybridisierung der Filter bei 42°C über Nacht in einer Denhardt-Lösung, 2 x SSC, 50 mM Natriumphosphat (pH 6,5), Kalbsthymus-DNA (250 µg/ml) und 35 % entionisiertes Formamid, danach Hybridisierung in der gleichen Lösung während 48 Stunden bei 42°C mit viraler DNA, die mit $^{32}$P (2 x 10$^4$ cpm/cm$^2$) markiert ist, und Waschen der Filter unter Bewegung während 30 Minuten bei 50°C in einer Lösung von 60 mM Tris-Chlorhydrat (pH 8), 2 mM EDTA, 300 mM NaCl und 0,5% Natriumdodecylsulfat, danach während 30 Minuten bei Raumtemperatur in einer Lösung von 3 mM Tris,

oder DNA-Fragmente, die einem der Bereiche E1, E6, E7, L1, L2 oder einem intergenischem Bereich dieser DNA-HPV entsprechen, oder ein DNA-Fragment, das einer DNA entspricht, die mit dem vorangehenden unter den angegebenen stringenten Bedingungen hybridisiert.

19. Verfahren zum Nachweis und zur Identifizierung des Papillomavirus eines Typs oder Subtyps, der einer DNA gemäß irgendeinem der Ansprüche 10 bis 17 entspricht, gegebenenfalls in einer biologischen Flüssigkeit enthalten, umfassend die Durchführung eines Hybridisierungstests mit der DNA oder einem entsprechenden DNA-HPV-Fragment, das als Sonde dient, und Nachweis des gegebenenfalls zwischen den DNA-HPVs der biologischen Flüssigkeit und der verwendeten Sonde gebildeten Hybrids.

# FIG.1

HPV 1a — Bam HI, Hind III, Pst I, Pst I, Pvu II, Sac I, Hind III, Bgl II, Eco RI, Pvu II, Bgl II, Hind II–Hpa I, Eco RI, Hind II–Hpa I, Bgl II, Bgl II, Bgl I, Hind III, Hind II

HPV 2d — Eco RI, Hind II, Hind II, Bg II, Pvu II, Pst I, Hind II–Hpa I, Hind II, Bam HI, Pvu II, Pst I, Bgl I, Bam HI, Pst I, Pvu II, Pvu II, Pst I

HPV 4a — Bam HI, Hind III, Pvu II, Hind II, Eco RI, Hind II, Hind II, Hind II, Eco RI, Bgl I, Hind II–Hpa I, Hind III

0  10  20  30  40  50  60  70  80  90  100

EP 0 192 001 B1

FIG.2

# FIG.3

EP 0 192 001 B1

Scale: 0 10 20 30 40 50 60 70 80 90 100

**HPV_5a** — Sac I, Hind II, Eco RI, Hind III, Hind II_Hpa I, Hind II_Hpa I, Hind II, Bgl I, Hind II_Hpa I, Pvu II, Bgl II, Bam HI, Ava I, Hind II, Hind II_Hpa I, Hind II, Pst I, Ava I, Ava I, Hind II_Sal I, Pst I, Pst I, Eco RI, Pvu II, Ava I, Bam HI, Bgl II, Pst I, Hind III

**HPV17a** — Bam HI ★, Ava I ★, Pvu II ★, Hind II ★, Bgl II ★, Hind II ★, Eco RI ★, Hind III, Bgl II, Pst I ★, Hind III ★, Eco RI ★, Pst I ★, Sac I ★, Hind II_Hpa I ★, Hind III ★, Eco RI ★, Hind II ★, Bgl II, Ava I ★, Pvu II, Pst I, Sac I, Pvu II ★, Hind III, Hind II ★, Hind III ★, Hind III ★

**HPV 24** — Bam HI, Hind III, Pvu II, Ava I, Hind II, Bgl II, Pst I, Hind III, Hind II, Bgl II, Hind II_Hpa I, Hind II_Hpa I, Pst I, Pst I, Bgl II, Pst I, Hind II, Hind II, Pst I, Hind III, Hind III, Pst I, Bam HI, Ava I, Pvu II, Bgl II, Hind II

FIG.4a

HPV_5a

HPV_8

HPV_12

HPV_16a
HPV_16b

FIG.4b

HPV_19 HPV_20 HPV_21 HPV_22 HPV_23

# FIG.5

FIG.6

FIG.7

FIG.8

HPV_IP 2

FIG. 9

HPV_IP 4

-BgI II (0)
- Hind III (3.8)
- Pst I (8.6)
- Kpn I (12.5)

Pvu II (22.5)
Pst I (23.8)
P st I (25.7)
Hind II (26.7)
- Hind III (29.8)
Xba I (30.2)

- Kpn I (40.4)

- BgI I (47.6)
- Pst I (51.4)

Pvu II (73.5)
- Hind II (74.3)
- Ava I - Sma I (76.9)

- Hind II - Npa I (85.7)

- Hind II - Npa I (89.5)

- X ba I (96.2)
- BgI II (100 )

0
10
20
30
40
50
60
70
80
90
100

Eco RI
Pst I
BgI I

Pst I
K pn I
Pst I

Kpn I

Pvu I

Hinc II
Pst I
Hinc II - Hpa I

Kpn I
Bam HI
Pst I
Ava I
Hinc II
Sac I
Pst I
Hind III
Pvu II
Hinc II
Bam HI